# EUROPEAN PATENT SPECIFICATION

(11) **EP 4 248 957 B1**
(45) Date of publication and mention of the grant of the patent: **09.09.2026**
(21) Application number: 22828609.2
(22) Date of filing: 13.05.2022
(51) Int. Cl.: A61K 31/12, A61K 31/444, A23L 33/10, A23L 33/105, A61K 45/06, A61P 1/16

(54) **CO-ADMINISTRATION OF CURCUMIN DERIVATIVE AND TGF-BETA RECEPTOR INHIBITOR FOR USE IN THE TREATMENT OF NON-ALCOHOLIC STEATOHEPATITIS**
CO-VERABREICHUNG VON CURCUMINDERIVATEN UND EINES TGF-BETA-REZEPTOR-INHIBITORS ZUR VERWENDUNG BEI DER BEHANDLUNG VON NICHTALKOHOLISCHER STEATOHEPATITIS
CO-ADMINISTRATION DE DÉRIVATIVES DE CURCUMINE ET D' INHIBITEUR DE RÉCEPTEUR TGF-BÉTA POUR UTILISATION DANS LE TRAITEMENT DE L' HÉPATITE STÉATOSIQUE NON ALCOOLIQUE

(30) Priority: 24.06.2021 KR 20210082417; 22.11.2021 KR 20210160931
(43) Date of publication of application: 27.09.2023
(73) Proprietor: University Industry Foundation, Yonsei University Wonju Campus, Gangwon-do 26493 (KR)
(72) Inventor: CHUNG, Choon Hee, Wonju-si Gangwon-do 26438 (KR); LEE, Eun Soo, Wonju-si Gangwon-do 26448 (KR); HA, Kyung Bong, Wonju-si Gangwon-do 26385 (KR); LEE, Dong Keon, Chuncheon-si Gangwon-do 24420 (KR); PARK, Na Won, Wonju-si Gangwon-do 26493 (KR); JO, Su Ho, Wonju-si Gangwon-do 26493 (KR)
(74) Representative: Vossius & Partner Patentanwälte Rechtsanwälte mbB
(86) International application number: PCT/KR2022/006873
(87) International publication number: WO 2022/270760

(56) References cited:
- WO-A1-2022/204721
- JP-A- 2007 320 864
- JP-A- 2007 320 864
- KR-A- 20140 104 090
- KR-A- 20140 104 090
- KR-B1- 101 927 399
- KR-B1- 101 927 399
- LEE EUN SOO ET AL: "Curcumin analog CUR5-8 ameliorates nonalcoholic fatty liver disease in mice with high-fat diet-induced obesity", METABOLISM, W.B. SAUNDERS CO., PHILADELPHIA, PA, US, vol. 103, 20 November 2019 (2019-11-20), XP086025513, ISSN: 0026-0495, [retrieved on 20191120], DOI: 10.1016/J.METABOL.2019.154015
- FARZAEI MOHAMMAD HOSEIN ET AL: "Curcumin in liver diseases: A systematic review of the cellular mechanisms of oxidative stress and clinical perspective", NUTRIENTS, 1 July 2018 (2018-07-01), pages 1 - 28, XP055817269, Retrieved from the Internet <URL:https://www.ncbi.nlm.nih.gov/pmc/articles/PMC6073929/pdf/nutrients-10-00855.pdf> [retrieved on 20210623]
- BAZIAR NIMA ET AL: "The effects of curcumin supplementation on body mass index, body weight, and waist circumference in patients with nonalcoholic fatty liver disease: A systematic review and doseresponse meta-analysis of randomized controlled trials", 4 December 2019 (2019-12-04), pages 464 - 474, XP093197434, Retrieved from the Internet <URL:https://pubmed.ncbi.nlm.nih.gov/31799714/> [retrieved on 20240821], DOI: 10.1002/ptr.6542
- ZHAO MENGYAO ET AL: "Current innovations in nutraceuticals and functional foods for intervention of non-alcoholic fatty liver disease", PHARMACOLOGICAL RESEARCH, ELSEVIER, AMSTERDAM, NL, vol. 166, 24 February 2021 (2021-02-24), XP086526053, ISSN: 1043-6618, [retrieved on 20210224], DOI: 10.1016/J.PHRS.2021.105517
- PARK SANG-A ET AL: "EW-7197 inhibits hepatic, renal, and pulmonary fibrosis by blocking TGF-[beta]/Smad and ROS signa", CMLS CELLULAR AND MOLECULAR LIFE SCIENCES, BIRKHAUSER VERLAG, HEIDELBERG, DE, vol. 72, no. 10, 9 December 2014 (2014-12-09), pages 2023 - 2039, XP035485829, ISSN: 1420-682X, [retrieved on 20141209], DOI: 10.1007/S00018-014-1798-6
- KIM MIN-JIN, PARK SANG-A, KIM CHUN HWA, PARK SO-YEON, KIM JUNG-SHIN, KIM DAE-KEE, NAM JEONG-SEOK, SHEEN YHUN YHONG: "TGF-β Type I Receptor Kinase Inhibitor EW-7197 Suppresses Cholestatic Liver Fibrosis by Inhibiting HIF1α-Induced Epithelial Mesenchymal Transition", CELLULAR PHYSIOLOGY AND BIOCHEMISTRY, KARGER BASEL, CH, vol. 38, no. 2, 1 January 2016 (2016-01-01), CH , pages 571 - 588, XP055776646, ISSN: 1015-8987, DOI: 10.1159/000438651
- PARK SANG-A, KIM MIN-JIN, PARK SO-YEON, KIM JUNG-SHIN, PARK MIN-KYUNG, KIM DAE-KEE, NAM JEONG-SEOK, SHEEN YHUN YHONG: "P1-90: Anti-fibrotic activity of EW-7197 in CC14-mice models of liver fibrosis by inhibition of TGF-β1/Smad signaling", THE FALL INTERNATIONAL CONVENTION OF THE PHARMACEUTICAL SOCIETY OF KOREA 2013; NOVEMBER 16 TO 17, 2013, PHARMACEUTICAL SOCIETY OF KOREA, KOREA, 1 January 2013 (2013-01-01) - 2013-11-17, Korea, pages 265, XP009543256
- MIN-JIN KIM; SANG-A PARK; SO-YEON PARK; JI YEON SON; SOL-JI KIM; SEON-JOO LEE; MIN-KYUNG PARK; DAE-KEE KIM; JEONG-SEOK NAM; YHUN Y: "P-HT-02: EW-7197, a novel orally available ALK5 inhibitor inhibits hepatic fibrosis in BDL-rat model by inhibition of TGF-β1/Smad signaling", PROCEEDINGS OF THE KOREA SOCIETY OF ENVIRONMENTAL TOXICOLOGY AUTUMN INTERNATIONAL CONFERENCE 2013 [ABSTRACTS]; OCTOBER 29-30, 2013, KOREA SOCIETY OF ENVIRONMENTAL TOXICOLOGY, KOREA, 1 January 2013 (2013-01-01) - 30 October 2013 (2013-10-30), Korea, pages 706, XP009543255

## Description

### [Technical Field]

The present invention relates to a pharmaceutical composition, or a combination kit for use in preventing, ameliorating, or treating metabolic liver diseases, comprising curcumin derivatives or pharmaceutically acceptable salts thereof, and transforming growth factor beta (TGF-β) receptor inhibitors or pharmaceutically acceptable salts thereof as active ingredients.

### [Background Art]

Examples of hepatic steatosis diseases include hepatic steatosis disease caused by alcohol, and metabolic liver diseases such as hepatic steatosis, steatohepatitis, or liver fibrosis caused by metabolic disorder.

A non-alcoholic fatty liver disease is a kind of the metabolic liver disease described above and is a disease that does not relate to alcohol consumption and is caused due to fat accumulation in liver. The non-alcoholic fatty liver disease means a series of disease groups including simple steatosis only exhibiting excessive accumulation of fat in hepatocytes, and non-alcoholic steatohepatitis (NASH) accompanied by hepatocyte necrosis, inflammation, and fibrosis.

The non-alcoholic steatohepatitis (NASH) is a disease occurring in the process of deterioration of non-alcoholic fatty liver diseases (NAFLD). First, inflammatory cytokines are secreted where destroyed hepatocyte fragments are phagocytosed by Kupffer cells and macrophages. The secreted cytokine activates hepatic stellate cells that regulate blood flow between hepatic sinusoid endothelial cells and hepatocytes to synthesize and secrete connective tissue components including collagen, thereby causing fibrosis. This process progresses not to steatosis with simple fatty metamorphosized hepatocytes but to a serious lesion of non-alcoholic steatohepatitis (NASH) that causes ballooning, inflammation, or fibrosis.

Meanwhile, curcumin (diferuloylmethane) is curcuminoid of turmeric, a yellow spice containing polyphenols, that is extracted from a root of *Curcuma longa* Linn, a plant belonging to the Zingiberaceae of East India, and is widely used in Indian food. Curcumin is used as a yellow pigment in food additives and used as a spice. Roles of curcumin in cellular physiology is diverse and involves many cellular components, including inflammation-related proteins, cell signaling materials, and transcription factors.

In addition, transforming growth factor-β (TGF-β) is a cytokine that transdifferentiates hepatic stellate cells (HSCs) into myofibroblasts to progress fibrosis in the liver, and thus blocking TGF-β signaling in a chronic liver disease is known as an ideal method for treating liver fibrosis. EW-7197, a TGF-β receptor inhibitor, is an inhibitor of TGF-β receptor AKL4(Activin receptor-like kinase 4) or AKL5 (Activin receptor-like kinase 5) that is highly potent and selective and can be orally administered. It has been found that EW-7197 has an effect of suppressing liver damage in a liver fibrosis model by administration of carbon tetrachloride (CCL4) and bile duct ligation in previous studies and also suppressing tissue fibrosis by regulating TGF-β/Smad and ROS signals in a model in which renal fibrosis was induced by unilateral ureteral obstruction (UUO) and lung fibrosis was induced by bleomycin (BLM).

However, TGF-β receptor inhibitors have side effects of promoting adipogenesis in the liver in the process of suppressing liver fibrosis, and thus a more effective treatment method for treating non-alcoholic steatohepatitis is required.

Accordingly, the present inventors have endeavored to develop a more effective treatment method for metabolic liver disease, in particular, non-alcoholic steatohepatitis, to newly find that an effect can be exhibited of suppressing fat accumulation in the liver together with suppressing liver fibrosis by performing co-administration of curcumin derivatives and TGF-β receptor inhibitors, and completed the present invention.

Non-Patent Literature 1 describes the benefit of the curcumin analog, curcumin 5-8, in a mouse model of nonalcoholic fatty liver disease. Patent Literature 1 discloses a pharmaceutical composition comprising a curcumin derivative for treating or preventing non-alcoholic fatty liver disease (NAFLD). Patent Literature 2 discloses the use of curcumin or a curcumin-containing product for treating non-alcoholic steatohepatitis. Non-Patent Literature 2 reviews the role of curcumin in liver diseases, namely non-alcoholic steatohepatitis (NASH), non-alcoholic liver disease, liver fibrosis and cirrhosis. Patent Literature 3 discloses the use of a pharmaceutical composition for the treatment and prevention of NAFLD comprising curcumin as an active ingredient. Non-Patent Literature 3 refers to a meta-analysis of randomized controlled trials treating NAFLD patients with curcumin or curcumin derivatives. Non-Patent Literature 4 summarizes the studies with curcumin in models of NAFLD. Patent Literature 4 shows the benefit of EW-7197 administration for treating liver fibrosis. Non-Patent Literature 5 discloses the benefit of the ALK-5 inhibitor EW-7197 treatment in different mouse models for fibrosis including liver fibrosis. However, none of the cited literatures discloses the combination of a curcumin derivative and a TGF-beta receptor inhibitor for use in treating metabolic liver diseases.

### [Citation List]

### [Patent Literature]

[Patent Literature 1]
   Korea Patent Registration No. 10-1927399
Non-Patent Literature 1: Lee et al., Metabolism Clinical and Experimental 103 (2020) 154015
Patent Literature 2: JP 2007-320864 A
Non-Patent Literature 2: Hosein et al., Nutrients 2018, 10, 855
Patent Literature 3: KR 2014-0104090 A
Non-Patent Literature 3: Nima et al., Phytotherapy Research. 2020;34:464-474
Non-Patent Literature 4: Mengyao et al., Pharmacological Research 166 (2021) 105517
Patent Literature 4: WO 2022/204721 A1
Non-Patent Literature 5: Park et al., Cell. Mol. Life Sci. (2015) 72:2023-2039

### [Summary of Invention]

### [Technical Problem]

Existing transforming growth factor-β (TGF-β) receptor inhibitors have anti-fibrotic effects but have a problem of increasing adipogenesis in hepatocytes in the process of suppressing tissue fibrosis.

An object of the present invention is to solve the above problems and to provide a pharmaceutical composition for use in preventing or treating a metabolic liver disease, the pharmaceutical composition comprising: a curcumin derivative or pharmaceutically acceptable salts thereof; and a TGF-β receptor inhibitor or pharmaceutically acceptable salts thereof as active ingredients.

Another object of the present invention is to provide a combination kit for use in treating a metabolic liver disease, the kit containing a preparation comprising a curcumin derivative or pharmaceutically acceptable salts thereof; and a TGF-β receptor inhibitor or pharmaceutically acceptable salts thereof as active ingredients, in combination.

### [Solution to Problem]

In order to achieve the object of the present invention, the present invention provides a pharmaceutical composition for use in preventing or treating a metabolic liver disease, the pharmaceutical composition comprising: a curcumin derivative or pharmaceutically acceptable salts thereof; and a TGF-β receptor inhibitor or pharmaceutically acceptable salts thereof as active **ingredients.**

According to the present invention, the curcumin derivative is at least one selected from the group consisting of N-{3-[(2E)-3-(4-hydroxy-3-methoxyphenyl)prop-2-enoyl]phenyl}-2-methylpropanamide, 4,4'-(3,5-pyridinediyldi-2,1-ethynediyl)bis(2-methoxyphenol), 2,6-dichloro-N-{3-[(2E)-3-(4-hydroxy-3-methoxyphenyl)-2-propenoyl]phenyl}benzamide, dehydrozingerone (DHZ), 4-(3,4-dimethoxyphenyl)-3-buten-2-one, and 1-(4-chlorophenyl) -3-phenyl-1,3-propanedione. **Examples of the curcumin derivative which do not form part of the claimed invention include demethoxycurcumin,** bisdemethoxycurcumin, 5-hydroxy-1-(4-hydroxy-3-methoxyphenyl)-7-(4-nitro-phenyl)-hepta-1,4,6-trien-3-one.

According to an aspect of the present invention, the composition can inhibit TGF-β receptor AKL4 (Activin receptor-like kinase 4) or AKL5 (Activin receptor-like kinase 5).

According to an aspect of **the** present invention, the TGF-β receptor inhibitor is N-(2-fluorophenyl)-5-(6-methyl-2-pyridinyl)-4-[1,2,4]triazolo[1,5-a]pyridin-6-yl-1H-imidazole-2-methanamine (EW-7197).

According to an aspect of the present invention, a molar ratio between a curcumin derivative and a TGF-β receptor inhibitor is 1:15 to 15:1.

According to an aspect of the present invention, the metabolic liver disease is at least one selected from the group consisting of hepatic steatosis, liver fibrosis, and steatohepatitis but is not limited thereto.

According to a specific aspect of the present invention, the steatohepatitis is non-alcoholic steatohepatitis.

In addition, the present invention provides an oral pharmaceutical preparation containing the pharmaceutical composition **for use in preventing or treating a metabolic liver** disease.

According to an aspect of the present invention, the oral pharmaceutical preparation includes at least one selected from the group consisting of tablets, granules, pills, powders, capsules, and liquids, but is not limited thereto.

In addition, **disclosed is** a quasi-drug for use in preventing or treating a metabolic liver disease, the quasi-drug comprising a curcumin derivative or pharmaceutically acceptable salts thereof; and a TGF-β receptor inhibitor or pharmaceutically acceptable salts thereof as active **ingredients.**

In addition, **disclosed is** a food composition for use in preventing or ameliorating a metabolic liver disease, the food composition comprising: a curcumin derivative or pharmaceutically acceptable salts thereof; and a TGF-β receptor inhibitor or pharmaceutically acceptable salts thereof as active **ingredients.**

In addition, the present invention provides a combination kit for use in treating a metabolic liver disease, the kit containing a preparation comprising: a curcumin derivative or pharmaceutically acceptable salts thereof; and a TGF-β receptor inhibitor or pharmaceutically acceptable salts thereof as active ingredients in **combination.**

### [Advantageous Effects of Invention]

An effect was confirmed, in which increase of fat accumulation in the liver, which is a side effect shown in case of administering a TGF-β receptor inhibitor singly, is inhibited when a curcumin derivative and a transforming growth factor-β (TGF-β) receptor inhibitor according to the present invention are combined, mixed, or sequentially administered. Therefore, a composition containing a curcumin derivative and a TGF-β receptor inhibitor as active ingredients can be usefully used for the purpose of preventing, ameliorating, or treating a metabolic liver disease.

### [Brief Description of Drawings]

FIG. 1 is a diagram showing an effect on cell survival when mouse hepatocyte cell lines are treated with TGF-β and various curcumin derivatives for the induction of fibrosis at different concentrations;
FIG. 2 is a diagram showing an effect of inhibiting liver fibrosis through protein expression of fibrosis markers when mouse hepatocyte cell lines in which fibrosis was induced by TGF-β were treated with EW-7197;
FIG. 3 is a diagram showing an effect of inhibiting liver fibrosis through protein expression of fibrosis markers when human hepatic stellate cell lines in which fibrosis was induced by TGF-β were treated with EW-7197;
FIG. 4 is a diagram showing a fat reduction effect of curcumin or a curcumin derivative after mouse hepatocyte cell lines were treated with oleic acid, an unsaturated fatty acid, so that fat is produced, and then treated with curcumin or a curcumin derivative;
FIG. 5 is a diagram showing a positively stained fat region in FIG. 4 in a digitizing manner;
FIG. 6 is a diagram showing fat synthesis and degradation after mouse hepatocyte cell lines were treated with oleic acid, an unsaturated fatty acid, so that fat is produced, and then treated with a curcumin derivative or EW-7197;
FIG. 7 is a diagram showing fat synthesis and degradation through protein expression of fat accumulation factors and fat metabolism regulators when mouse hepatocyte cell lines were treated with oleic acid, an unsaturated fatty acid, so that fat is produced, and then treated with a curcumin derivative or EW-7197;
FIG. 8 is a diagram showing a synergistic effect of a treatment with EW-7197 and curcumin 5-8 in combination and is a drawing showing an effect of inhibiting epithelial-mesenchymal conversion, an effect of inhibiting adipogenesis in hepatocytes, and an effect of inhibiting liver fibrosis when hepatocytes in which fibrosis was induced by TGF-β were treated with EW-7197 and curcumin 5-8 in combination;
FIG. 9 is a diagram showing a positively stained fat region in FIG. 8 in a digitizing manner;
FIG. 10 is a diagram showing a positively stained a-SMA region in FIG. 8 in a digitizing manner;
FIG. 11 is a diagram showing a synergistic effect when mouse hepatocyte cell lines were treated with EW-7197 and curcumin 5-8 in combination and is a diagram showing an effect of inhibiting liver fibrosis through protein expression of fibrosis markers when hepatocyte cell lines in which fibrosis was induced by TGF-β were treated with EW-7197 and curcumin 5-8 in combination;
FIG. 12 is a diagram showing a synergistic effect when human hepatic stellate cell lines were treated with EW-7197 and curcumin 5-8 in combination and is a diagram showing an effect of inhibiting liver fibrosis through protein expression of fibrosis markers when hepatocyte cell lines in which fibrosis was induced by TGF-β were treated with EW-7197 and curcumin 5-8 in combination;
FIG. 13 is a diagram showing a synergistic effect when mouse hepatocyte cell lines were treated with 10 µM or 0.5 µM EW-7197 and curcumin 5-8 in combination and is a diagram showing an effect of inhibiting adipogenesis in hepatocytes when hepatocyte in which fibrosis was induced by TGF-β were treated with EW-7197 and Curcumin 5-8 in combination;
FIG. 14 is a diagram showing a positively stained fat region of a 10 µM EW-7197 treated group in FIG. 13 in a digitizing manner;
FIG. 15 is a diagram showing a positively stained fat region of a 0.5 µM EW-7197 treated group in FIG. 13 in a digitizing manner;
FIG. 16 is a diagram showing a synergistic effect when mouse hepatocyte cell lines were treated with EW-7197 and curcumin 5-8, dehydrozingerone (DHZ), dehydrozingerone 103 (DHZ 103), or dehydrozingerone 176 (DHZ 176) in combination and is a diagram showing an effect of inhibiting epithe_ial-mesenchymal conversion, an effect of inhibiting adipogenesis in hepatocytes, and an effect of inhibiting liver fibrosis when hepatocytes in which fibrosis was induced by TGF-β were treated with EW-7197 and curcumin 5-8, DHZ, DHZ 103, or DHZ 176 in combination;
FIG. 17 is a diagram showing a positively stained fat region in FIG. 16 in a digitizing manner;
FIG. 18 is a diagram showing a positively stained a-SMA region in FIG. 16 in a digitizing manner;
FIG. 19 is a diagram showing physiological changes when an animal model in which steatohepatitis was induced with a methionine-choline deficient diet (MCD) was treated with high-concentration EW-7197 (40 mg/kg) and curcumin 5-8 in combination;
FIG. 20 is a diagram showing an effect of inhibiting fat accumulation in liver tissues and tissue fibrosis when the animal model of FIG. 19 was treated with a high-concentration EW-7197 (40 mg/kg) and curcumin 5-8 in combination and a diagram showing fat droplets and fibrosis levels of liver tissues by Hematoxylin & Eosin (H&E), Picrosirius red, Masson's trichrome staining;
FIG. 21 is a diagram showing a positively stained Picrosirius red and Masson's trichrome regions in FIG. 20 in a digitizing manner;
FIG. 22 is a diagram showing the evaluation of the H & E-stained liver tissue in FIG. 20 with a NAFLD activity score (NAS) system;
FIG. 23 is a diagram showing an effect of inhibiting fat accumulation in liver tissues through protein expression of fat metabolism regulators and signaling factors related thereto when the animal model of FIG. 19 is treated with high-concentration EW-7197 (40 mg/kg) and curcumin 5-8 in combination;
FIG. 24 is a diagram showing an effect of inhibiting fibrosis of liver tissues through protein expression of fibrosis markers when the animal model of FIG. 19 was treated with high-concentration EW-7197(40mg/kg) and curcumin 5-8 in combination;
FIG. 25 is a diagram showing physiological changes when an animal model in which steatohepatitis was induced with an MCD was treated with low-concentration EW-7197 (5 mg/kg) and curcumin 5-8 in combination;
FIG. 26 is a diagram showing an effect of inhibiting fat accumulation in liver tissues and tissue fibrosis when the animal model of FIG. 25 was treated with low-concentration EW-7197 (5 mg/kg) and curcumin 5-8 in combination and is a diagram showing fat droplets and fibrosis levels of liver tissues by Hematoxylin & Eosin (H&E), Picrosirius red, Masson's trichrome staining;
FIG. 27 is a diagram showing a positively stained Picrosirius red and Masson's trichrome regions in FIG. 26 in a digitizing manner; and
FIG. 28 is a diagram showing the evaluation of the H & E-stained liver tissue in FIG. 26 with a NAFLD activity score (NAS) system.

### [Description of Embodiments]

Hereinafter, embodiments of the present invention are described in detail so that those skilled in the art can easily implement the present invention. The embodiments of the present invention are provided to more completely explain the present invention to those ordinarily skilled in the art. Therefore, the embodiments of the present invention can be modified in many different forms, and the scope of the present invention is not limited to the embodiments described below.

Throughout the specification of the present invention, when it is said that a part "includes" a certain component, it means that the part may further include other components without excluding other components unless otherwise stated.

As used herein, the expression "pharmaceutically acceptable" means that the contained components do not significantly stimulate living organisms and do not interfere with biological activities and properties.

As used herein, "pharmaceutically acceptable salts" refer to salts having desired biological activities, and examples thereof include inorganic acid salts (hydrochloric acid, sulfuric acid, phosphoric acid, and nitric acid), organic acid salts (acetic acid, oxalic acid, maleic acid, fumaric acid, succinic acid, benzoic acid, ascorbic acid, tannic acid, pamoic acid, alginic acid, triethylamine, cyclohexylamine, and pyridine), alkali metal salts (sodium salts and potassium salts), alkaline earth metal salts (calcium salts), ammonium salts, and addition salts thereof, but are not limited thereto.

In the present specification, the expression "prevention" refers to all activities that inhibit symptoms or progress of a specific disease (for example, a metabolic liver disease) by introducing the composition of the present invention into the body.

In the present invention, the expression "treatment" refers to all activities that improve or beneficially change symptoms of a specific disease (for example, a metabolic liver disease) by injecting the composition of the present invention into the body.

The present invention provides a pharmaceutical composition for use in preventing or treating a metabolic liver disease, the pharmaceutical composition comprising: a curcumin derivative or pharmaceutically acceptable salts thereof; and a TGF-β receptor inhibitor or pharmaceutically acceptable salts thereof as active **ingredients.**

According to the present invention, curcumin refers to polyphenol mainly extracted from a root of *Curcuma longa* Linn.

In the present invention, a curcumin derivative refer to a compound in which a part of a chemical structure of curcumin, which is a kind of polyphenol, is deleted, added, or changed. Examples of the curcumin derivative **which do not form part of the claimed invention include demethoxycurcumin,** bisdemethoxycurcumin, 5-hydroxy-1-(4-hydroxy-3-methoxyphenyl)-7-(4-nitro-phenyl)-hepta-1,4,6-trien-3-one, **and the curcumin derivative of the claimed invention is at least one selected from N-{3-**[(2E)-3-(4-hydroxy-3-methoxyphenyl)prop-2-enoyl]phenyl}-2-methylpropanamide, 4,4'-(3,5-Pyridinediyldi-2,1-ethynediyl)bis(2-methoxyphenol), 2,6-dichloro-N-{3-[(2E)-3-(4-hydroxy-3-methoxyphenyl)-2-propenoyl]phenyl}benzamide, Dehydrozingerone (DHZ), 4-(3,4-Dimethoxyphenyl)-3-buten-2-one, and 1-(4-Chlorophenyl)-3-phenyl-1,3-propanedione.

According to an aspect of the present invention, the curcumin derivative of the present invention may be N-{3-[(2E)-3-(4-hydroxy-3-methoxyphenyl)prop-2-enoyl]phenyl}-2-methylpropanamide and may have the structure of [Formula 1]. In an example of the present invention, the curcumin derivative refers to curcumin 5-8 (CUR5-8).

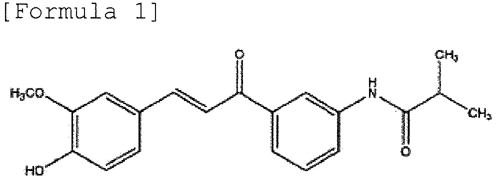

In addition, the curcumin derivative of the present invention may be 4,4'-(3,5-Pyridinediyldi-2,1-ethynediyl)bis(2-methoxyphenol) and may have the structure of [Formula 2]. In an example of the present invention, the curcumin derivative refers to curcumin 4-8 (CUR4-8).

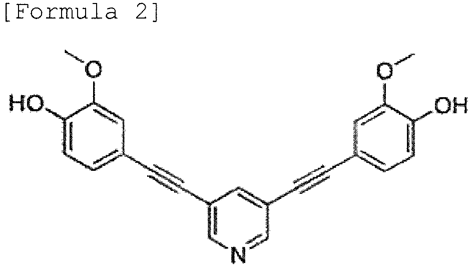

In addition, the curcumin derivative of the present invention may be 2,6-Dichloro-N-{3-[(2E)-3-(4-hydroxy-3-methoxyphenyl)-2-propenoyl]phenyl}benzamide and may have the structure of [Formula 3]. In an example of the present invention, the curcumin derivative refers to Curcumin 5-14 (CUR5-14).

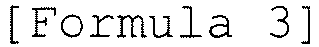

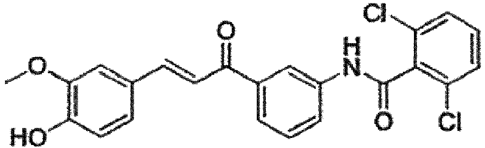

In addition, the curcumin derivative of the present invention may be dehydrozingerone (DHZ) and may have the structure of [Formula 4].

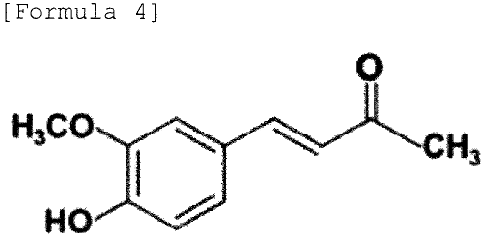

In addition, the curcumin derivative of the present invention may be 4-(3,4-Dimethoxyphenyl)-3-buten-2-one and may have the structure of [Formula 5]. In an example of the present invention, the curcumin derivative refers to dehydrozingerone 103 (DHZ 103).

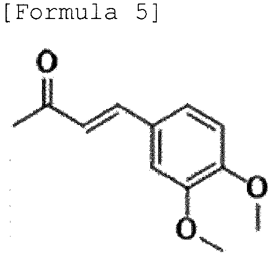

In addition, the curcumin derivative of the present invention may be 1-(4-Chlorophenyl)-3-phenyl-1,3-propanedione and may have the structure of [Formula 6]. In an example of the present invention, the curcumin derivative refers to dehydrozingerone 176 (DHZ 176).

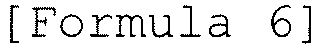

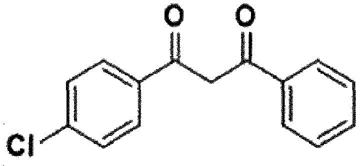

The curcumin derivative of the present invention may exhibit an effect of inhibiting adipogenesis by a TGF-β receptor inhibitor in hepatocytes.

The curcumin derivative of the present invention may be synthesized according to known methods and used, or commercially available curcumin derivative (for example, available from Sigma-Aldrich) may be purchased and used.

According to an aspect of the present invention, the TGF-β receptor inhibitor may be N-(2-fluorophenyl)-5-(6-methyl-2-pyridinyl)-4-[1,2,4]triazolo[1,5-a]pyridin-6-yl-1H-imidazole-2-methanamine and may have the structure of [Formula 7]. In an example of the present invention, the TGF-β receptor inhibitor refers to EW-7197.

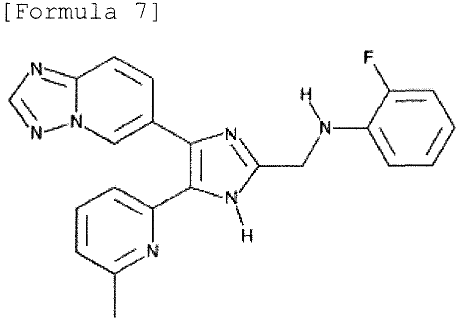

In the present invention, EW-7197 can be orally administered, inhibit a liver damage in a liver fibrosis model by CCL4 administration and bile duct ligation, and inhibit tissue fibrosis in a renal fibrosis model by unilateral ureteral obstruction (UUO) and a lung fibrosis model by bleomycin.

According to an aspect of the present invention, the pharmaceutical composition can inhibit a TGF-β receptor AKL4 or AKL5.

The TGF-β receptor inhibitor of the present invention may be synthesized according to known methods and used, or commercially available TGF-β receptor inhibitors may be purchased and used.

The molar ratio between the curcumin derivative and the TGF-β receptor inhibitor of the present invention may be 1:15 to 15:1, 1:14 to 14:1, 1:13 to 13:1, 1:12 to 12:1, 1:11 to 11:1, or 1:10 to 10:1.

According to a specific aspect of the present invention, the curcumin derivative may be curcumin 5-8, dehydrozingerone, dehydrozingerone 103, or dehydrozingerone 176, and the TGF-β receptor inhibitor may be EW-7197.

The metabolic liver disease according to the present invention may be at least one selected from the group consisting of hepatic steatosis, steatohepatitis, and liver fibrosis. Specifically, the metabolic liver disease of the present invention may be steatohepatitis, and specifically non-alcoholic steatohepatitis. The non-alcoholic steatohepatitis of the present invention may be accompanied by fatty metamorphosis of hepatocytes, necrosis, inflammation, and fibrosis.

In addition, the pharmaceutical composition of the present invention not only may include active ingredients of the present invention but also may further include a substance previously recognized to have an effect of treating a hepatic steatosis disease, an effect of treating a liver disease, an effect of protecting the liver, and an effect of ameliorating the function of the liver, for example, a hepatic steatosis disease treatment agent, a substance for protecting hepatocytes, and substances to be used as a liver disease treatment agent or a liver function enhancing agent or may be used together with the above substances. When the pharmaceutical composition of the present invention is used together with hepatic steatosis preventing or treating agents in the related art, the pharmaceutical composition and the agents can be administered simultaneously or sequentially, and the administration is not affected by the frequency and order.

The content of the curcumin derivative or the pharmaceutically acceptable salts thereof; and the TGF-β receptor inhibitor or the pharmaceutically acceptable salts thereof in the pharmaceutical composition of the present invention may be 0.001 wt% to 99.999 wt%, 0.01 wt% to 99.99 wt%, 0.1 wt% to 99.9 wt%, or 1 wt% to 90 wt%, and is not limited thereto. The content of the curcumin derivative or the pharmaceutically acceptable salts thereof and the TGF-β receptor inhibitor or the pharmaceutically acceptable salts thereof according to the present invention can be appropriately adjusted and used according to a subject of administration, a mode of use, a method of use, a form of the preparation, and the like.

In addition, the pharmaceutical composition of the present invention is administered in a pharmaceutically effective amount.

As used in the present invention, the expression "pharmaceutically effective amount" means an amount sufficient to treat a disease with a reasonable benefit/risk ratio applicable to medical treatment, and the effective amount level can be determined according to factors including the type and severity of the patient's disease, drug activity, drug sensitivity, administration time, an administration route and an excretion rate, the duration of treatment, and drugs used concurrently, and other factors well known in the medical field. The pharmaceutical composition according to the present invention may be administered as an individual treating agent, may be administered in combination with surgery, hormone treatment, drug treatment, and biological response modifiers, may be administered simultaneously, separately, or sequentially with the above preparations, and may be administered singly or multiply. Considering all of the above factors, it is important to administer an amount that can obtain the maximum effect with the minimum amount without side effects, which can be easily determined by those skilled in the art.

Specifically, the effective amount of the pharmaceutical composition of the present invention may vary depending on the age, the sex, the condition, and the body weight of a patient, absorption rates, inactivation rates, and excretion rates of active ingredients in the body, the disease type, and combined drugs. Generally, 0.1 mg to 100 mg and preferably 0.3 mg to 80 mg per 1 kg of the body weight may be administered daily or every other day or may be administered by being divided into once to 3 times a day. However, since the administration amount may increase or decrease depending on the route of administration, the severity of obesity, the gender, the weight, the age, and the like, the administration amount is not limited to the scope of the present invention in any way.

The pharmaceutical composition of the present invention may be administered orally or parenterally (for example, intramuscularly, intravenously, intraperitoneally, subcutaneously, intradermally, or locally applied) depending on the desired method, and the administration amount varies according to the condition and the body weight of the patient, the severity of disease, the drug form, and the route and duration of administration but can be appropriately selected by those skilled in the art.

In addition, the pharmaceutical composition of the present invention can be delivered using a pharmaceutically acceptable carrier such as colloidal suspension, powder, saline solution, lipids, liposomes, microspheres, or nano-spherical particles. The pharmaceutical composition may form a complex with a vehicle, may be linked to each other, may be included in a carrier, and may be delivered into/in the body by using delivery systems known in the art such as lipids, liposomes, microparticles, gold, nanoparticles, polymers, condensation reagents, polysaccharides, polyamino acids, dendrimers, saponins, adsorption enhancing substances, or fatty acids. In addition, pharmaceutically acceptable carriers may include lactose, dextrose, sucrose, sorbitol, mannitol, starch, acacia, rubber, calcium phosphate, alginate, gelatin, calcium silicate, microcrystalline cellulose, polyvinyl pyrrolidone, cellulose, water, syrup, methyl cellulose, methyl hydroxy benzoate, propyl hydroxy benzoate, talc, magnesium stearate, and mineral oil which are generally used during the preparation but are not limited thereto. In addition to the above components, lubricants, wetting agents, sweeteners, flavoring agents, emulsifiers, suspending agents, preservatives, and the like may be further included.

In addition, when formulating the pharmaceutical composition of the present invention, the formulation may be performed be using diluents or excipients such as commonly used fillers, extenders, binders, wetting agents, disintegrants, and surfactants. Examples of solid preparations for oral administration include tablets, pills, powders, granules, and capsules, and these solid preparations may be manufactured by mixing at least one excipient (starch, calcium carbonate, sucrose, lactose, or gelatin) and a lubricant (magnesium stearate talc) or the like. Examples of liquid preparations for oral administration include suspensions, internal solutions, emulsions, or syrups, and these liquid preparations may be manufactured by mixing at least one of diluent (water and liquid paraffin), excipients, wetting agents, sweeteners, flavors, preservatives, and the like. Examples of preparations for parenteral administration include sterilized aqueous solutions, non-aqueous solvents, suspension solutions, emulsions, freeze-dried preparations, and suppositories. As the non-aqueous solvents and suspension solutions, propylene glycol, polyethylene glycol, vegetable oil such as olive oil, and injectable esters such as ethyl oleate may be used. As the suppositories, Witepsol, Macrogol, Tween 61, cacao butter, laurin butter, glycerol, gelatin, and the like may be used.

According to an aspect of the present invention, the pharmaceutical composition for preventing or treating a metabolic liver disease may be an oral pharmaceutical preparation. Specifically, the oral pharmaceutical preparation may include at least one selected from the group consisting of tablets, granules, pills, powders, capsules, and liquids.

According to an aspect of the present invention, the preparations for oral administration may be present, for example, in dosage unit form and ampoules such as tablets, granules, pills, powders, capsules or solutions. The preparations are manufactured by known methods, for example, general mixing, granulating, confectioning, formulating, dissolving, or lyophilizing methods.

**Disclosed is also a quasi-drug for use in** preventing or treating a metabolic liver disease, the quasi-drug comprising: a curcumin derivative or pharmaceutically acceptable salts thereof; and a TGF-β receptor inhibitor or pharmaceutically acceptable salts thereof as active **ingredients.**

The curcumin derivative or the pharmaceutically acceptable salts thereof; the TGF-β receptor inhibitor or the pharmaceutically acceptable salts thereof, and the metabolic liver disease of the present **disclosure** are the same as those described with respect to the pharmaceutical composition for **use in preventing or treating a** metabolic liver disease, the pharmaceutical composition comprising: a curcumin derivative or pharmaceutically acceptable salts thereof; and a TGF-β receptor inhibitor or pharmaceutically acceptable salts thereof as active ingredients. Therefore, the above contents are referred, and hereinafter only the unique configuration of quasi-drugs is described.

As used in the present **disclosure,** the expression "quasi-drugs" refers to items with a milder effect than pharmaceuticals among items used for the purpose of diagnosing, treating, ameliorating, mitigating, treating or preventing diseases of humans or animals. For example, according to the Pharmaceutical Affairs Act, the quasi-drugs are items excluding items used for pharmaceutical purposes and may be products used for treating or preventing diseases in humans or animals or products with minor or no direct action on the human body.

The quasi-drug composition can be manufactured in a formulation selected from the group consisting of a body cleanser, a disinfectant cleanser, a cleaning agent, a kitchen detergent, a cleaning detergent, toothpaste, gargle, a wet tissue, a detergent, a soap, hand wash, hair wash, hair softener, a humidifier filler, a mask, ointment, a filter filler, and an oral preparation converted from a pharmaceutical such as a vitamin preparation, a mineral preparation, a nutrient altering agent, a gastric digestive agent, and an intestinal agents. Specifically, the quasi-drug may be an internal medicine converted from a pharmaceutical drug.

Disclosed is a food composition for use in preventing or ameliorating a metabolic liver disease, the food composition comprising: a curcumin derivative or pharmaceutically acceptable salts thereof; and a TGF-β receptor inhibitor or pharmaceutically acceptable salts thereof as active **ingredients.**

The curcumin derivative or the pharmaceutically acceptable salts thereof; the TGF-β receptor inhibitor or pharmaceutically acceptable salts thereof; and the metabolic liver disease according to the present **disclosure** are the same as those described with respect to the pharmaceutical composition for **use in preventing or treating** a metabolic liver disease, the pharmaceutical composition comprising: the curcumin derivative or the pharmaceutically acceptable salts thereof; and the TGF-β receptor inhibitor or the pharmaceutically acceptable salts thereof as active ingredients. Therefore, the above contents are referred, and hereinafter only the unique configuration of the food composition is described.

The food composition may be a functional food or health functional food and may be a health functional food composition for ameliorating or preventing a metabolic liver disease and further protecting the liver and improving the liver function.

The food composition refers to a natural product or processed product containing one or more nutrients, for example, may mean an item that became directly edible through a certain degree of processing process, and usually includes all food, food additives, and health functional foods and beverages.

Examples of the food to which the curcumin derivative or the pharmaceutically acceptable salts thereof; and the TGF-β receptor inhibitor or the pharmaceutically acceptable salts thereof may be added include various kinds of foods, beverages, gum, tea, vitamin complexes, and functional foods. Additionally, the foods

include special nutritional food (for example, formula milk, infant food, and baby food), processed meat products, fish meat products, tofu, jellied foods, noodles (for example, ramen and noodles), health supplement foods, seasoning foods (for example, soy sauce, soybean paste, red pepper paste, and mixed paste), sauces, confectioneries (for example, snacks), dairy products (for example, fermented milk and cheese), other processed foods, kimchi, pickled foods (various types of kimchi, pickles, and the like), drinks (for example, fruit and vegetable beverages, soymilk, and fermented beverages), and natural seasonings (for example, a ramen soup), but the food is not limited thereto. The foods, beverages, or food additives may be manufactured by a manufacturing method in the related art.

The functional food refers to a food group in which an added value is added to the food by using physical, biochemical, or bioengineering methods so that the function of the food acts or is expressed for a specific purpose; or food designed and processed to sufficiently express the body control function of the food composition relating to biological defense rhythm control, disease prevention and recovery, and the like in the living body. The functional food may include food additives that are acceptable in food science and may further include appropriate carriers, excipients, and diluents commonly used in the manufacture of functional foods.

In the present disclosure, a beverage refers to a general term for drinking tc quench thirst or enjoy taste and includes functional beverages. The beverage has no particular limitation on the inclusion of other ingredients other than including the curcumin derivative or the pharmaceutically acceptable salts thereof; and the TGF-β receptor inhibitor or the pharmaceutically acceptable salts thereof as active ingredients, as essential components in the indicated ratio and may contain various flavoring agents or natural carbohydrates as additional ingredients as in general beverages. Examples of the natural carbohydrates include conventional sugars of monosaccharides such as glucose and fructose; disaccharides such as maltose and sucrose; polysaccharides such as dextrins and cyclodextrins, and sugar alcohols such as xylitol, sorbitol, and erythritol. As flavoring agents other than those mentioned above, natural flavoring agents (thaumatin, stevia extract (for example, rebaudioside A and glycyrrhizin), and synthetic flavoring agents (saccharin, aspartame, and the like) can advantageously be used. The ratio of the natural carbohydrates is generally about 1 g to 20 g, or 5 g to 12 g per 100 ml of the composition of the present disclosure. In addition, the composition may further contain fruit flesh for manufacturing natural fruit juice, fruit juice beverages, and vegetable beverages.

Other than the above, the food composition may contain various nutrients, vitamins, minerals (electrolytes), flavors such as synthetic flavors and natural flavors, colorants and enhancers (such as cheese and chocolate), pectic acid and salts thereof, alginic acid and salts thereof, organic acids, protective colloidal thickeners, pH adjusters, stabilizers, preservatives, glycerin, alcohol, carbonation agents used in carbonated beverages, and the like. These components may be used independently or in combination. The proportion of these additives is not so critical but may be selected in the range of 0 parts by weight to 200,000 parts by weight per 100 parts by weight of the food composition of the present **disclosure.**

In the present **disclosure,** the functional beverage refers to a group of beverages in which an added value is added to the beverage by using physical, biochemical, or bioengineering methods so that the function of the beverage acts or is expressed for a specific purpose; and beverages designed and processed to sufficiently express the body control function of the beverage composition relating to biological defense rhythm control, disease prevention, and recovery, and the like in the living body.

The functional beverage has no particular limitation on the inclusion of other ingredients other than including the active ingredient of the food composition of the present **disclosure** as essential components essential components in the indicated ratio and may contain various flavoring agents or natural carbohydrates as additional ingredients as in general beverages. Examples of the natural carbohydrates include conventional sugars of monosaccharides such as glucose and fructose; disaccharides such as maltose and sucrose; polysaccharides such as dextrins and cyclodextrins, and sugar alcohols such as xylitol, sorbitol, and erythritol. As flavoring agents other than those mentioned above, natural flavoring agents (thaumatin, stevia extract (for example, rebaudioside A and glycyrrhizin), and synthetic flavoring agents (saccharin, aspartame, and the like) can advantageously be used. The ratio of the natural carbohydrates is generally about 1 g to 20 g, or 5 g to 12 g per 100 ml of the composition of the present **disclosure.**

In addition, in the food composition for ameliorating or preventing a metabolic liver disease, the food composition comprising the curcumin derivative or the pharmaceutically acceptable salts thereof; and the TGF-β receptor inhibitor or the pharmaceutically acceptable salts thereof as active ingredients, the content of the active ingredient is 0.0001 wt% to 99.99 wt%, 0.001 wt% to 99.9% wt%, 0.01 wt% to 90 wt%, or 0.1 wt% to 50 wt%, with respect to the total weight of the food. In the beverage composition, the active ingredient may be contained in the proportion of 0.0002 g to 5 g, or 0.03 g to 1 g based on 100 ml.

In addition, the intake amount of the food composition for ameliorating or preventing a metabolic liver disease, the food composition comprising the curcumin derivative or the pharmaceutically acceptable salts thereof; and the TGF-β receptor inhibitor or the pharmaceutically acceptable salts thereof as active ingredients, varies depending on the condition and weight of the intake subject, the degree of disease, the type of drug, and the route and duration of the administration, but can be appropriately selected by those skilled in the art. For example, the food composition can be administered at 0.0001 g/kg (amount of active ingredients/body weight) to 12 g/kg (amount of active ingredients/body weight) or at 0.01 g/kg to 9 g/kg per day based on the active ingredient. As the administration method, the administration may be performed once a day or divided into several times, and the administration frequency and administration method **are not limited** in any way.

The food composition can be formulated in the same way as the pharmaceutical composition and used as a functional food or can be added to various foods. Examples of the foods to which the composition of the present **disclosure** can be added include beverages, meats, chocolates, foods, confectioneries, pizza, ramen, other noodles, chewing gum, ice cream, alcoholic beverages, vitamin complexes, and health supplements.

Also, the present invention provides a combination kit for use in treating a metabolic liver disease, the kit containing a preparation comprising a curcumin derivative or pharmaceutically acceptable salts thereof; and a TGF-β receptor inhibitor or pharmaceutically acceptable salts thereof as active ingredients, in **combination.**

The curcumin derivative or the pharmaceutically acceptable salts thereof; the TGF-β receptor inhibitor or pharmaceutically acceptable salts thereof; and the metabolic liver disease according to the present invention are the same as those described with respect to the pharmaceutical composition for **use in preventing or treating** a metabolic liver disease, the pharmaceutical composition comprising the curcumin derivative or the pharmaceutically acceptable salts thereof; and the TGF-β receptor inhibitor or the pharmaceutically acceptable salts thereof as active ingredients. Therefore, the above contents are referred.

Hereinafter, the present invention is described in detail through examples and experimental examples.

### <Example 1> Cell Experiment

### <Example 1-1> Cell Culture

Cell experiments were performed by using AML12, rat hepatocytes, and LX-2 cells, a human hepatic stellate cell.

AML12 cells were cultured in a culture medium made by adding 5 ml of insulin (ITS), 40 ng/ml of dexamethasone, 1% penicillin streptomycin (anti-anti), and 10% fetal bovine serum (FBS) to F-12 media (Corning Incorporated, USA, Arizona), a mixture of a DMEM medium and Ham's F-12 at a ratio of 50:50, which is used for culturing hepatocytes or prostate epithelial cells.

LX-2 cells were cultured in a culture medium made by adding 1% penicillin streptomycin (anti-anti) and 10% fetal bovine serum (FBS) to a DMEM medium containing high glucose (4500 mg/L D-glucose, L-glutamine, 110 mg/L sodium pyruvate, sodium bicarbonate).

### <Example 1-2> Confirmation of Cytotoxicity of Curcumin Derivatives

In order to confirm cytotoxicity, the cells of Example 1-1 were treated with 2 ng/ml of TGF-β, and then with curcumin (CUR), curcumin 5-8 (CUR5-8), curcumin 4-8 (CUR4-8), and curcumin 5-14 (CUR5-14) at 0.1, 1, 3, and 5 µM, respectively, for the same period of time. Thereafter, cells cultured in an incubator of 5% CO₂ at 37°C were stained with a tetrazolium dye capable of staining surviving cells (MTT), and absorbance was measured by using a spectrophotometer.

### <Example 1-3> Confirmation of Fibrosis of Hepatocytes after Administration of TGF-β Receptor Inhibitor

Fibrosis was induced in hepatocytes with TGF-β, a TGF-β receptor inhibitor was administered, and fibrosis was checked.

Specifically, 2 ng/ml of TGF-β was administered to the cells of Example 1-1, and administered or not administered with 10 µM EW-7197 (EW), and cultured for 24 hours. The control group (Con) was not treated with either TGF-β or the sample. Thereafter, the cells were collected, and protein expressions of α-SMA, Collagen 1 (Coll), p-SMAD2, and p-SMAD3 as fibrosis markers in the cells were checked by Western blotting. To this end, the collected cells were lysed in RIPA buffer (Thermo, MA, USA) with protease inhibitors and phosphatase inhibitors (Roche, IN, USA) and then incubated on ice for 30 minutes. The lysate was centrifuged at 4°C at 13,000 rpm for 20 minutes to remove insoluble matters. A protein concentration was measured by BCA assay (Pierce Biotechnology Inc). The cell lysate in the equal amount was loaded on an SDS-PAGE gel for electrophoresis and transferred to PVDF membrane (polyvinylidene difluoride, Immobilon-P, Merck KGaA, Darmstadt, Germany). Then, the membranes were blocked with 5% (w/v) skim milk in 1X TBST for one hour at room temperature. After blocking, the membrane was treated with, as primary antibodies, anti-β-actin antibody (1:2000; Santa Cruz Biotechnology, Inc), anti-α-SMA antibody (1:1000; Santa Cruz Biotechnology, Inc), anti-collagen I antibody (1:1000; Santa Cruz Biotechnology, Inc), anti-p-SMAD2 antibody (1:1000; Cell Signaling Technology), and anti-p-SMAD3 antibody (1:1000; Cell Signaling Technology), respectively, and incubated at 4°C overnight. Then, after washing several times with 1X TBST, the membranes were incubated with horseradish peroxidase-conjugated secondary antibody (anti-mouse, rabbit or goat, 1:2000; Cell Signaling Technology) in blocking buffer for one hour at room temperature (RT). Then, the membranes were washed, briefly incubated with Pico Enhanced Peroxidase Detection (EPD) Western reagent (ELPISBIOTECH. INC.) according to the manufacturer's procedure, and quantified by using an image analyzer (ImageQuant^{™} LAS 500, GE Healthcare Bio-Sciences AB, Uppsala, Sweden). β-actin was used as an internal control. Densitometry of protein intensity was quantified by using ImageJ (National Institutes of Health).

### <Example 1-4> Confirmation of Fat Synthesis and Degradation after Curcumin Derivative Administration

Fat accumulation was induced in hepatocytes with oleic acid, curcumin derivatives were administered, and fat synthesis and degradation were confirmed.

Specifically, oleic acid was dissolved in 0.2% bovine serum albumin (BSA) at a concentration of 500 µM, and the cells of Example 1-1 were cultured for 24 hours and treated with the resultant, and also treated with 1 µM curcumin (Cur), 1 µM curcumin 5-8 (Cur5-8), 1 µM curcumin 4-8 (Cur4-8), and 1 µM curcumin 5-14 (Cur5-14) for the same period of time. Thereafter, BODIPY-fatty acid solution (Sigma, 1 mg/mL of stock DMSO solution was diluted with PBS 1X to obtain a final concentration of 0.2 µg/mL) was administered to cells cultured in an incubator of 5% CO₂ at 37°C and cultured for one hour. After washing with PBS 1X, stained cells were observed.

In addition, the cells of Example 1-1 were treated with oleic acid in the same manner as described above and treated with 1 µM curcumin (Cur), 1 µM Curcumin 5-8 (Cur5-8), and 10 µM EW-7197 (EW), and then BODIPY-fatty acid staining was performed in the same manner as above. In addition, the cells were collected after treatment, and the protein expressions of adipophilin as a fat accumulation inducing factor and SREBP1C as a fat metabolism regulator in the collected cells were checked by Western blotting. To this end, Western blotting was performed by using the collected cells in the same method as described in <Example 1-3>. At this time, an anti-β-actin antibody, an anti-adipophilin antibody, and an anti-SREBP1C antibody were used as primary antibodies.

### <Example 1-5> Confirmation of Fibrosis of Hepatocytes and Fat Synthesis and Degradation After Co-Administration of Curcumin Derivative and TGF-β Receptor Inhibitor

Fibrosis was induced in hepatocytes with TGF-β, a curcumin derivative and a TGF-β receptor inhibitor were co-administered, and then fibrosis and fat synthesis and degradation were checked.

Specifically, 2 ng/ml of TGF-β was administered to the cells of Example 1-1, the cells were divided into administration groups of vehicle (Veh), 1 µM curcumin (Cur), 1 µM curcumin 5-8 (Cur5-8), 0.5 µM EW-7197 (EW0.5), 10 µM EW-7197 (EW or EW10), 1 µM curcumin and 0.5 µM EW-7197 (EW0.5+Cur), 1 µM curcumin and 10 µM EW-7197 (EW+Cur or EW10+Cur), 1 µM curcumin 5-8 and 0.5 µM EW-7197 (EW0.5+Cur5-8), 1 µM curcumin 5-8 and 10 µM EW-7197 (EW+Cur5-8 or EW10+Cur5-8), respective samples were administered, and culturing was performed for 24 hours. The control group (Con) was not treated with either TGF-β or the sample. Subsequently, the cells cultured in an incubator of 5% CO₂ at 37°C were observed with a microscope to check epithelial-mesenchymal conversion, and BODIPY-fatty acid solution (Sigma, 1 mg/mL of stock DMSO solution was diluted with PBS 1X to obtain a final concentration of 0.2 µg/mL) was administered and culturing was performed for one hour. After washing with PBS 1X, stained cells were observed.

In addition, in order to check the degree of fibrosis of hepatocytes, the cells cultured for 24 hours were fixed with 4% paraformaldehyde and then stained by using α-SMA (Santa Cruz Biotechnology, Inc., Dallas, Texas, USA), an Alexafluor 488 (Cell Signaling Technology, Danvers, Massachusetts, USA) antibody, and DAPI (Abcam plc., Cambridge, MA, USA). The cells were mounted with a mounting solution (Fisher Scientific, Hampton, Rockingham, USA), the manufactured sample was photographed using a confocal microscope to obtain photographs of stained cells, and the degree of color development in the photographs of the stained cells was analyzed with the ImageJ program.

In addition, in order to confirm the degree of fibrosis of hepatocytes, the cells cultured for 24 hours were collected, and protein expressions of α-SMA, Collagen I (Col1), p-SMAD2, and p-SMAD3 were checked by Western blotting with fibrosis markers in the same method as described in <Example 1-3>.

### <Example 1-6> Confirmation of Fibrosis of Hepatocytes and Fat Synthesis and Degradation after Co-Administration of Dehydrozingerone Derivative and TGF-β Receptor Inhibitor

Fibrosis was induced in hepatocytes with TGF-β, dehydrozingerone or a derivative thereof as a curcumin derivative and a TGF-β receptor inhibitor were co-administered, and then fibrosis and fat synthesis and degradation were checked. In addition, the curcumin derivative Cur5-8 of Example 1-4 and the TGF-β receptor inhibitor were co-administered, and then fibrosis and fat synthesis and degradation were checked again.

Specifically, 2 ng/ml of TGF-β was administered to the cells of Example 1-1, the cells were divided into administration groups of vehicle (Veh), 10 µM EW-7197 (EW), 1 µM curcumin and 10 µM EW-7197 (EW+Cur), 1 µM curcumin 5-8 and 10 µM EW-7197 (EW+Cur5-8), 10 µM dehydrozingerone (DHZ) and 10 µM EW-7197 (EW+DHZ), 10 µM dehydrozingerone 103 (DHZ103) and 10 µM EW-7197 (EW+Z103), and 10 µM dehydrozingerone 176 (DHZ176) and 10 µM EW-7197 (EW+Z176), respective samples were administered, and the culturing was performed for 24 hours. The control group (Con) was not treated with either TGF-β or the sample. Subsequently, the cells cultured in an incubator of 5% CO₂ at 37°C were observed with a microscope to check epithelial-mesenchymal conversion, and BODIPY-fatty acid solution (Sigma, 1 mg/mL of stock DMSO solution was diluted with PBS 1X to obtain a final concentration of 0.2 µg/mL) was administered and culturing was performed for one hour. After washing with PBS 1X, stained cells were observed.

In addition, in order to check the degree of fibrosis of hepatocytes, the cells cultured for 24 hours were fixed with 4% paraformaldehyde and stained by using α-SMA (Santa Cruz Biotechnology, Inc., Dallas, Texas, USA), an Alexafluor 488 (Cell Signaling Technology, Danvers, Massachusetts, USA) antibody, and DAPI (Abcam plc., Cambridge, MA, USA). The cells were mounted with a mounting solution (Fisher Scientific, Hampton, Rockingham, USA), the manufactured sample was photographed using a confocal microscope to obtain photographs of stained cells, and the degree of color development in the photographs of the stained cells was analyzed with the ImageJ program.

### <Example 2> Animal Testing

### <Example 2-1> Manufacturing of Steatohepatitis Animal Model and Co-Administration of Curcumin Derivative and High-Concentration TGF-β Receptor Inhibitor

8-week-old male C57BL/6 mice weighing 20 to 25 g were purchased from Dae Han Bio Link Co., Ltd. (Chungbuk). Mice were acclimatized for one week under room temperature (25°C ± 2°C) in 60% ± 5% humidity and a 12-hour light/dark cycle. Mice were fed with a normal diet (Research Diets, RD) and allowed to drink water freely. Afterwards, the mice were randomly divided into five groups according to the fed diets or administered drugs, and the five groups were a group which was fed with a normal diet (Con), a group which was fed with a methionine-choline deficient diet (MCD), a group which was fed with a mixture of an MCD and curcumin 5-8 (1 g of curcumin 5-8 per 1 kg of the diet) (MCD+Cur5-8), a group which was fed with an MCD and to which EW-7197 was orally administered (MCD+EW), and a group which was fed with a mixture of an MCD and curcumin 5-8 and to which EW-7197 was orally administered together (MCD+EW+Cur5-8) (n = 10).

EW-7197 was administered directly to the stomach by an oral administration method once every two days at 40 mg/kg. Body weights were measured every week during the experiment period, and liver tissues were harvested after 6 weeks and stored at -80°C. All experiments were performed with the approval of the Institutional Animal Care and Use Committee at Wonju College of Medicine of Yonsei University (YWC-200907-1).

### <Example 2-2> Manufacturing of Steatohepatitis Animal Model and Co-Administration of Curcumin Derivative and Low-Concentration TGF-β Receptor Inhibitor

A steatohepatitis animal model was prepared in the same method as described in <Example 2-1>, and a curcumin derivative and EW-7197 were co-administered with the administration concentration of EW-7197 was 5 mg/kg.

### <Example 2-3> Confirmation of Physiological Changes in Steatohepatitis Animal Models

In order to confirm the physiological changes in case of co-administration of a curcumin derivative (Cur5-8) and a TGF-β receptor inhibitor (EW-7197), the liver weights and the concentrations of TG, TC, AST, ALT, and γ-GT in serum were measured. In addition, hydroxyprolin expression was checked as a fibrosis marker in the liver tissues.

Specifically, the weights of the liver tissues harvested in Examples 2-1 and 2-2 were measured before storage.

In Examples 2-1 and 2-2, blood was collected after the experiment was completed, and concentrations of TG, TC, AST, ALT, and γ-GT in serum were measured. In order to measure blood glucose, triglyceride, cholesterol, and AST and ALT concentrations, ELISA kit of Asan Pharm. Co., Ltd. was purchased, and the measuring was performed. γ-GT in the liver was measured by using Mak089 of Sigma-Aldrich, and hydroxyproline was measured by using STA-675 ELISA kit of Cell Biolabs, Inc.

In addition, the hydroxyprolin expression was checked in the liver tissues harvested in Examples 2-1 and 2-2.

### <Example 2-4> Observation of Liver Tissue Lesions in Steatohepatitis Animal Model

In order to check effects of inhibiting fat accumulation in liver tissues and fibrosis in liver tissues in case of co-administration of a curcumin derivative (CurS-8) and a TGF-β receptor inhibitor (EW-7197), the liver tissues were stained.

The liver tissues harvested in Examples 2-1 and 2-2 were fixed with 10% formaldehyde, the formaldehyde was washed out with running water for more than 12 hours, and then washing was performed with 60% ethanol at a one-hour interval, with 70% ethanol at a one-hour interval, and with 100% ethanol at a one-hour interval. Then, the transparency process in xylene was performed three times for one hour each, and the infiltration process in paraffin was performed twice for one hour each. Then, the paraffin block was cut to a thickness of about 4 µm to obtain slices. After deparaffinization, the slices were stained with hematoxylin and eosin (H&E staining), and blood vessels and lipid droplets in the liver tissues were checked.

After the liver tissue slices were obtained by the above method and stained with Picrosirius red and Masson's trichrome staining methods, tissue fibrosis around the blood vessels was checked by a microscope with a camera with a magnification of 400 times (Pulnix Sensors Inc., Sunnyvale, CA, USA).

In addition, H&E-stained liver slices were evaluated by using the NAFLD activity score (NAS) system. The system took into account the degrees of steatosis (0 to 3), lobular inflammation (0 to 3), and hepatocyte ballooning (0 to 2). The NAS score, which is the sum of these scores, scores less than 3 as being normal and 5 or more as being non-alcoholic steatohepatitis (NASH) to indicate the severity of NAFLD.

### <Example 2-5> Confirmation of Fibrosis and Fat Synthesis and Degradation in Liver Tissues of Steatohepatitis Animal Models

In order to check effects of inhibiting fat accumulation in liver tissues and fibrosis of liver tissues in case of co-administration of a curcumin derivative (Cur5-8) and a TGF-β receptor inhibitor (EW-7197), expressions of fibrosis markers and fat metabolism regulators in the liver tissues and signaling factors relating thereto were checked.

Specifically, Western blotting was performed by using the liver tissues harvested in Example 2-1 in the same method as described in <Example 1-3>. At this time, in order to check the protein expression of α-SMA, Coll, Fibronectin, p-SMAD2, and p-SMAD3 as fibrosis markers in the liver tissues, an anti-β-actin antibody, an anti-α-SMA antibody, an anti-Coll antibody, an anti-Fibronectin antibody, an anti-p-SMAD2 antibody, and an anti-p-SMAD3 antibody were used as primary antibodies. In addition, in order to check the protein expressions of Rock1, p-AMPK, AMPK, and SREBP1C as fat metabolism regulators and signaling factors relating thereto, an anti-β-actin antibody, an anti-Rock1 antibody, and an anti-p-AMPK antibody were used as primary antibodies.

### <Example 3> Statistical Analysis

All data were presented as mean ± standard deviation. Statistical analysis included one-way ANOVA and Tukey's post hoc test for multiple comparisons and was performed by using SPSS Statistics software (version 20.0; IBM, Armonk, NY, USA). Statistical significance was set at P < 0.05.

### <Experimental Example 1> Confirmation of Effects of Co-Administration of Curcumin Derivative and TGF-β Receptor Inhibitor in Mouse Hepatocyte Cell Lines or Human Hepatic Stellate Cell Lines

### <Experimental Example 1-1> Confirmation of Effects of Curcumin Derivative on Cells when Fibrosis was Induced by TGF-β in Mouse Hepatocyte Cell Lines

By a method described in Example 1-2, the numbers of surviving cells when curcumin (CUR) and curcumin derivatives (CUR5-8, CUR4-8, and CUR5-14) each were co-administered with TGF-β at respective concentrations were measured.

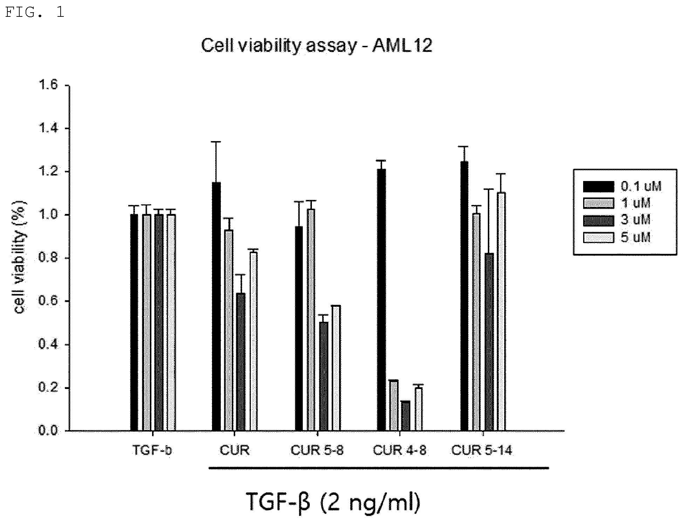

As a result, as shown in FIG. 1, it was confirmed that cell viability was the highest at concentrations of 0.1 µM and 1 µM, and cytotoxicity was high in the CUR4-8 treatment group at concentrations other than 0.1 µM.

### <Experimental Example 1-2> Confirmation of Effect of TGF-β Receptor Inhibitor on Fibrosis when Fibrosis was Induced by TGF-β in Mouse Hepatocyte Cell Lines or Human Hepatic Stellate Cell Lines

Fibrosis was induced with TGF-β by the method described in Examples 1-3, treated with a TGF-β receptor inhibitor (EW-7197), and protein expressions of α-SMA, Coll, p-SMAD2, and p-SMAD3 as fibrosis markers were checked by Western blotting.

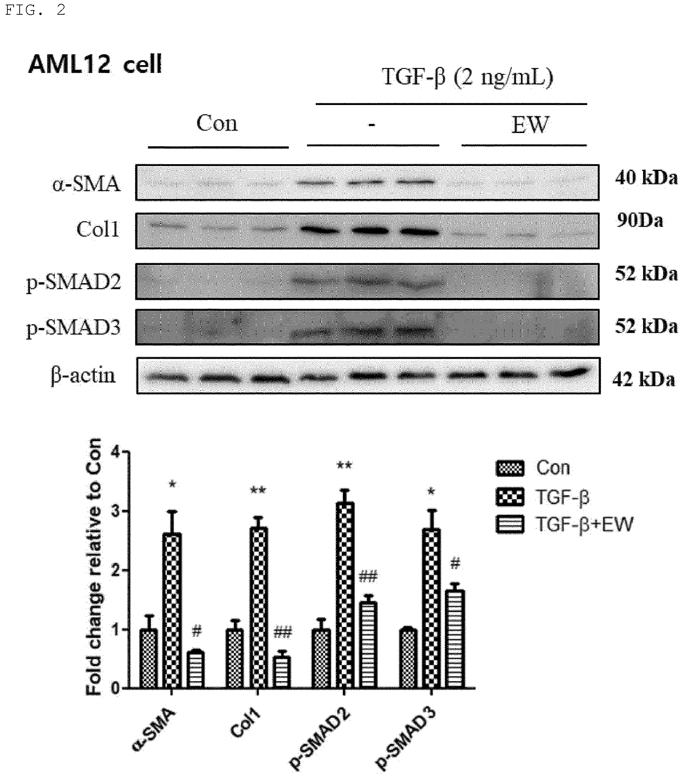

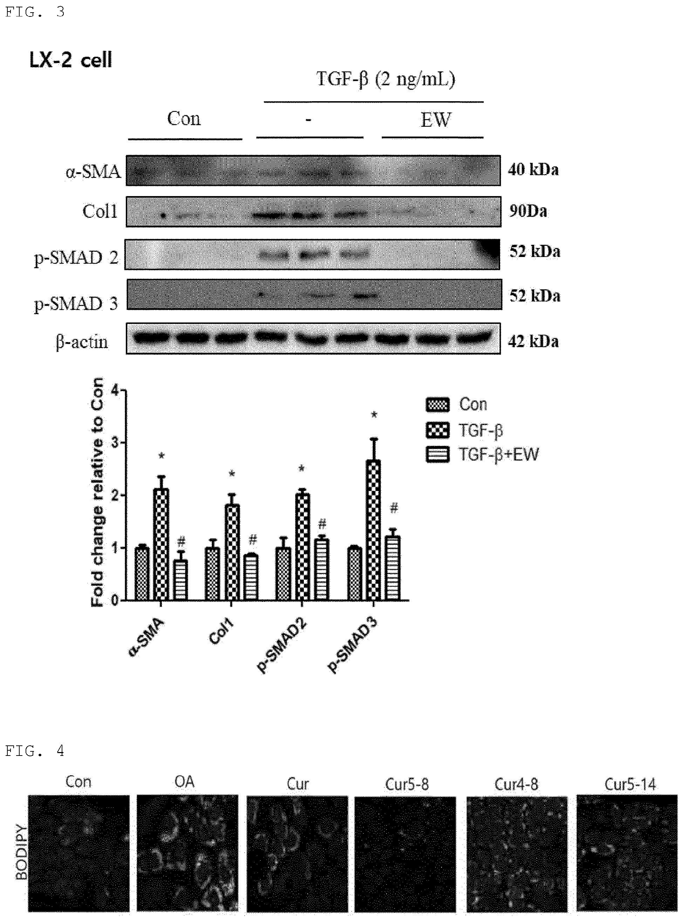

As a result, as shown in FIGS. 2 and 3, it was confirmed that protein expressions of α-SMA, Coll, p-SMAD2, and p-SMAD3 increased by TGF-β were inhibited by EW-7197 administration. Accordingly, it can be understood that liver fibrosis was inhibited by EW-7197 administration.

### <Experimental Example 1-3> Confirmation of Effects of Curcumin Derivative or TGF-β Receptor Inhibitor on Fat Synthesis When Fat Accumulation was Induced by Oleic Acid in Mouse Hepatocyte Cell Lines

The mouse hepatocyte cell lines were treated with oleic acid by the method described in Example 1-4 and were simultaneously treated with each of curcumin (Cur) and curcumin derivatives (Cur5-8, Cur4-8, and Cur5-14) for 24 hours, and fat was stained by BODIPY staining method.

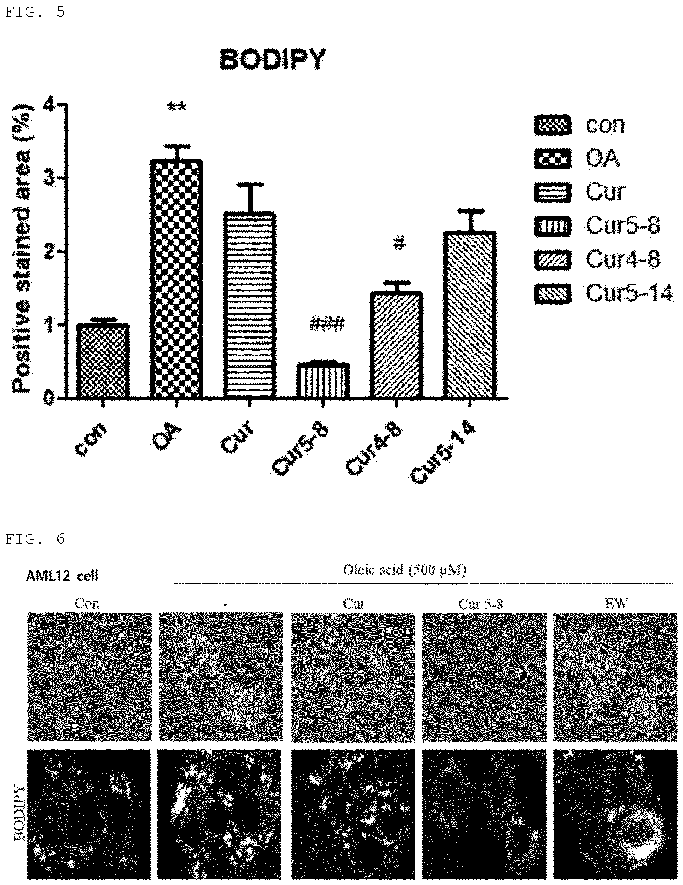

As a result, as shown in FIGS. 4 and 5, it was confirmed that curcumin derivatives reduced fat increased by oleic acid, and in particular, Cur5-8 most effectively reduced fat.

In addition, mouse hepatocyte cell lines were treated with oleic acid by the method described in Examples 1-4 and were simultaneously treated with curcumin (Cur), a curcumin derivative (Cur5-8), and a TGF-β receptor inhibitor (EW-7197), respectively, for 24 hours, fat was stained by BODIPY staining method, and Western blotting was performed.

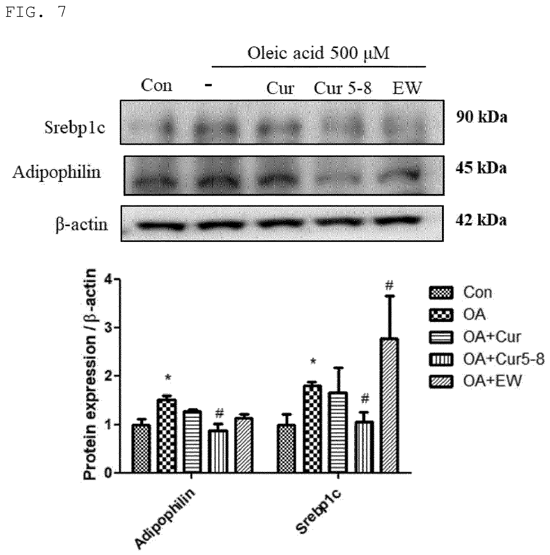

As a result, as shown in FIGS. 6 and 7, the curcumin derivative (Cur5-8) reduced the fat increased by oleic acid, while the TGF-β receptor inhibitor (EW-7197) promotes the fat increase caused by oleic acid.

### <Experimental Example 1-4> Confirmation of Inhibition of Fat Accumulation and Fibrosis by Curcumin Derivatives and TGF-β Receptor Inhibitor in Mouse Hepatocyte Cell Lines or Human Hepatic Stellate Cell Lines

In order to check a synergistic effect of inhibiting fibrosis caused by a TGF-β and inhibiting fat accumulation caused thereby in case of co-administration of a curcumin derivative (Cur5-8) and a TGF-β receptor inhibitor (EW-7197), epithelial-mesenchymal conversion of hepatocytes was confirmed through a cell experiment by the method described in Example 1-5, and BODIPY staining and α-SMA staining were performed. Also, protein expressions of α-SMA, Collagen I p-SMAD2, and p-SMAD3 were checked by Western blotting.

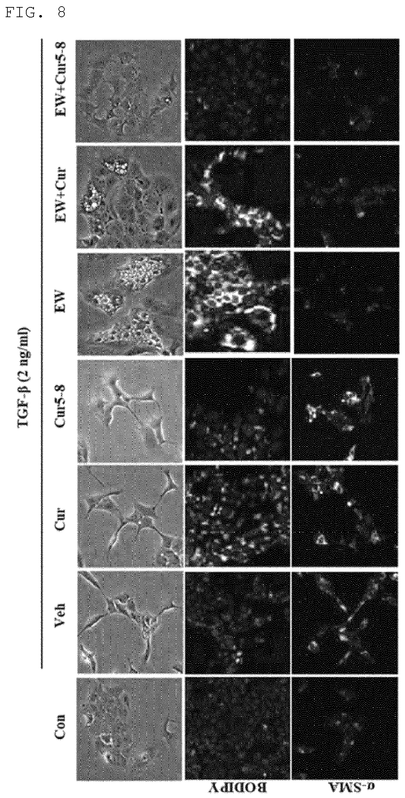

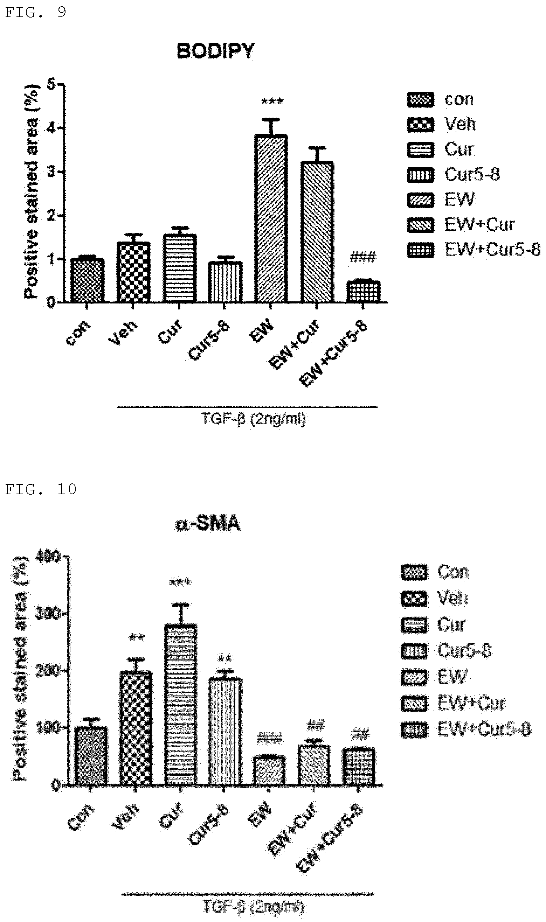

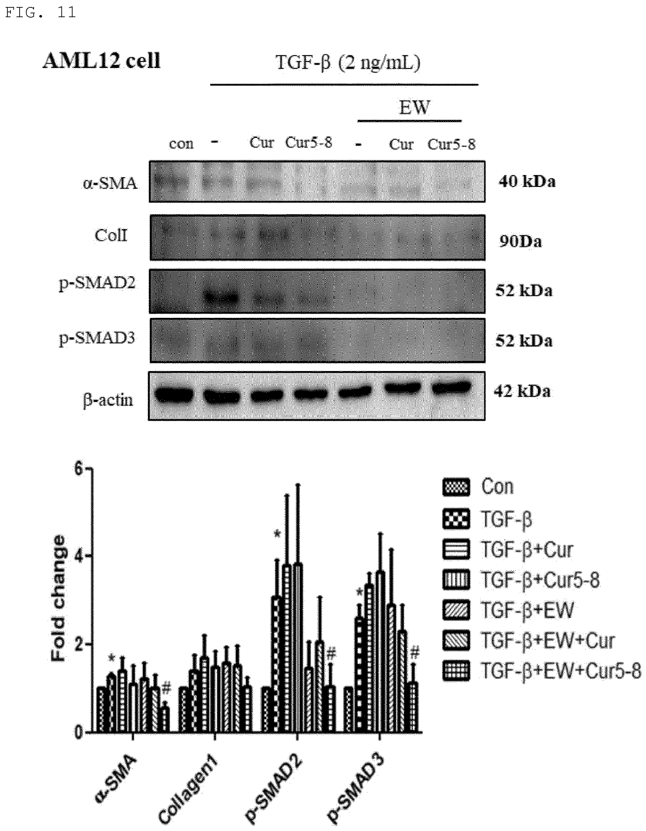

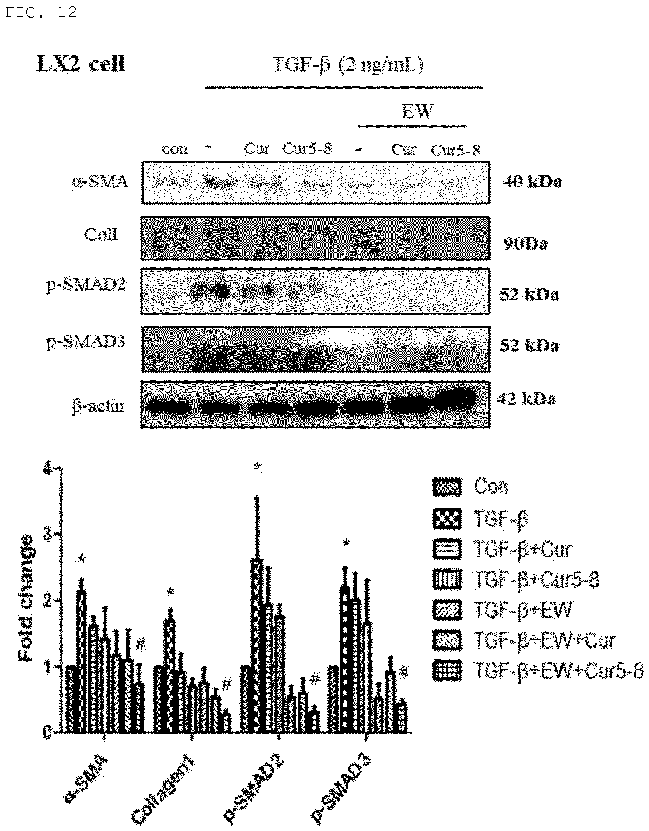

As a result, as shown in FIGS. 8 to 12, it was confirmed that, when hepatocytes were treated with 2 ng/ml of TGF-β for 24 hours, the morphology of hepatocytes was changed due to epithelial-mesenchymal conversion, and α-SMA staining confirmed that of hepatic fibrosis was induced (Veh group, green). It was confirmed that, also in the curcumin administration group (Cur) and the curcumin derivative administration group (Cur5-8), the epithelial-mesenchymal conversion caused by TGF-β was not inhibited, and liver fibrosis was induced. It was confirmed that, in the EW-7197 administration group (EW), the epithelial-mesenchymal conversion and liver fibrosis were inhibited, but BODIPY staining (red) confirmed that the effect of fat accumulation in cells was exhibited. Meanwhile, it was confirmed that, in the curcumin and EW-7197 co-administration group (EW+Cur) or the curcumin derivative and EW-7197 co-administration group (EW+Cur5-8), epithelial-mesenchymal conversion and liver fibrosis were inhibited, and adipogenesis in cells caused by EW-7197 was inhibited. In particular, it was confirmed that adipogenesis in the cells were inhibited more effectively in the EW+Cur5-8 group than in the EW+Cur group.

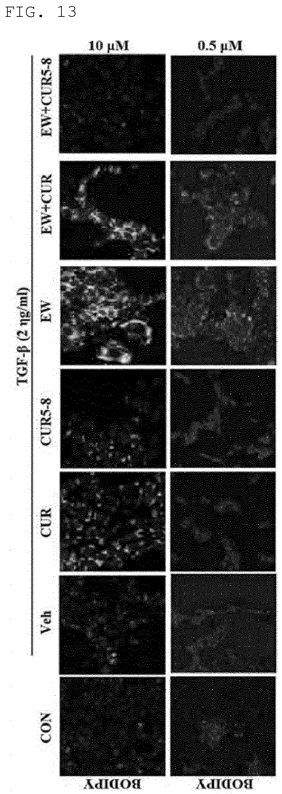

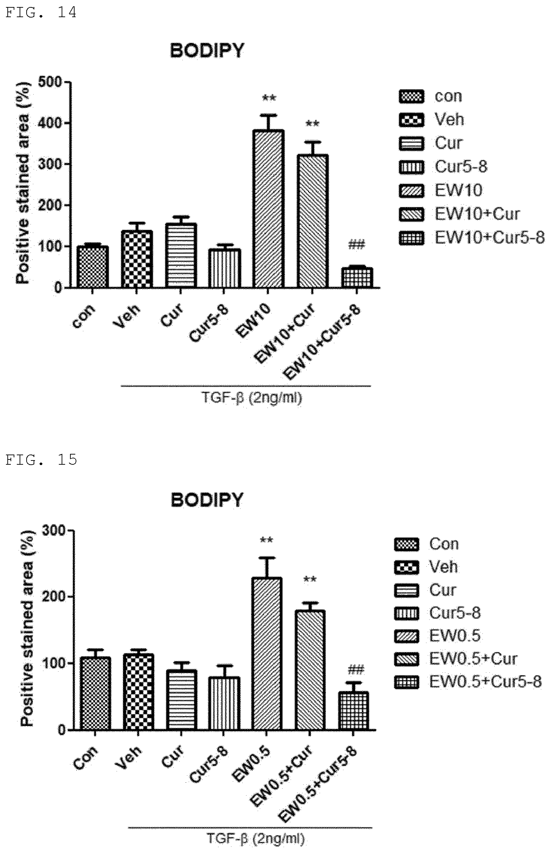

In addition, as shown in FIGS. 13 to 15, it was confirmed that, in all cases of high-concentration (10 µM) EW-7197 administration and low-concentration (0.5µM) EW-7197 administration, adipogenesis in cells was inhibited in case of co-administration of curcumin or a curcumin derivative, and adipogenesis in cells was inhibited more effectively in the curcumin derivative co-administration group.

### <Experimental Example 1-5> Confirmation of Effect of Inhibiting Fat Accumulation and Fibrosis by Dehydrozingerone Derivative and TGF-β Receptor Inhibitor in Mouse Hepatocyte Cell Lines

In order to check a synergistic effect of inhibiting fibrosis caused by TGF-β and inhibiting fat accumulation caused thereby in case of co-administration of dehydrozingerone (DHZ) as a curcumin derivative or a derivative thereof (DHZ103, DHZ176) with a TGF-β receptor inhibitor (EW-7197), the epithelial-mesenchymal conversion of hepatocyte was confirmed through a cell experiment by the method described in Example 1-6, and BODIPY staining and α-SMA staining were performed.

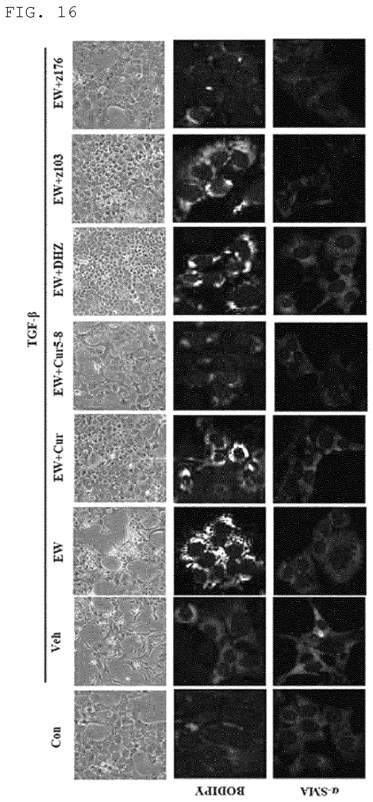

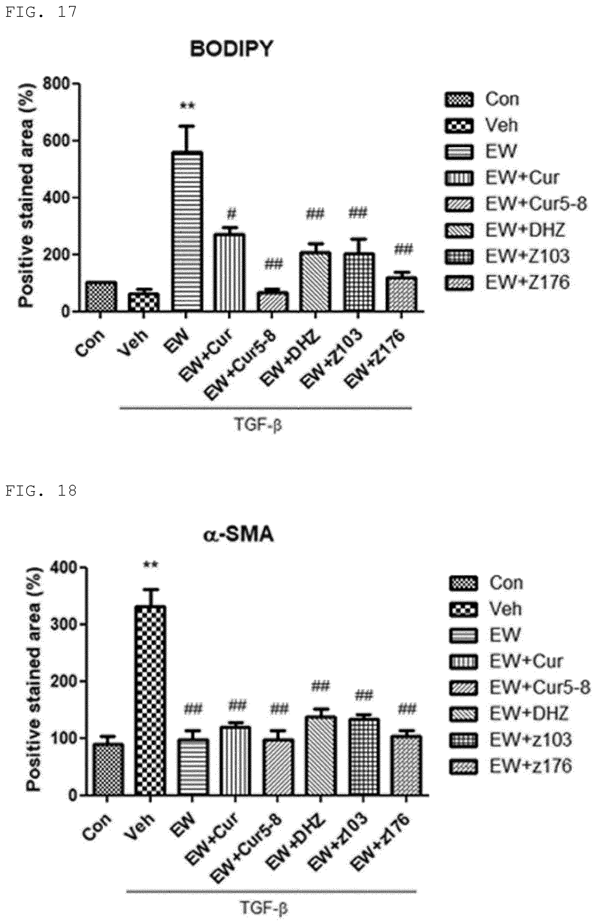

As a result, as shown in FIGS. 16 to 18, it was confirmed that, when hepatocyte cell lines were treated with 2 ng/ml of TGF-β for 24 hours, the morphology of hepatocytes was changed due to epithelial-mesenchymal conversion, and α-SMA staining confirmed that hepatic fibrosis was induced (Veh group, green). Also, in the EW-7197 administration group (EW), the epithelial-mesenchymal conversion and liver fibrosis were inhibited, but BODIPY staining (red) confirmed the effect of fat accumulation in cells was exhibited. Meanwhile, it was confirmed that, also in the co-administration group of dehydrozingerone or a derivative thereof with EW-7197 (EW+DHZ, EW+Z103, or EW+Z176), together with the curcumin and EW-7197 co-administration group (EW+Cur) and the Cur5-8 and EW-7197 co-administration group (EW+Cur5-8), it was confirmed that epithelial-mesenchymal conversion and liver fibrosis were inhibited and that adipogenesis in cells caused by EW-7197 was inhibited. It was confirmed that the adipogenesis in cells were inhibited more effectively in the EW+DHZ, EW+Z103, and EW+Z176 groups, than in the EW+Cur group.

### <Experimental Example 2> Confirmation of Effect of Inhibiting Liver Fibrosis and Fat Accumulation of Curcumin Derivative and TGF-β Receptor Inhibitor in Animal Model in which Steatohepatitis was Induced

### <Experimental Example 2-1> Confirmation of Effect of Inhibiting Liver Fibrosis and Fat Accumulation of Curcumin Derivative and High-Concentration TGF-β Receptor Inhibitor in Animal Model in which Steatohepatitis was induced.

In order to check the effect of inhibiting fibrosis in liver tissues and fat accumulation in liver tissues in case of co-administration of curcumin derivative (Cur5-8) and high-concentration TGF-β receptor inhibitor (EW-7197), an animal model in which steatohepatitis was induced was manufactured by the method described in Example 2-1, and the curcumin derivative (Cur5-8) and the high-concentration TGF-β receptor inhibitor (40 mg/kg EW-7197) were co-administered. Thereafter, the physiological changes were checked by the method described in Example 2-3, liver tissue lesion was observed by the method described in Example 2-4, and fibrosis and fat synthesis and degradation in the liver tissues were checked by the method described in Example 2-5.

**[Table 1]**

| Group | BW^{a} (g) | Liver weight/ Body weight*100(g) | TG^{b} (mg/dL) | TC^{c} (mg/dL) | AST^{d} (IU/L) | ALT^{e} (IU/L) |
|---|---|---|---|---|---|---|
| Con 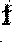 | 27.5±1.87 | 4.9±0.27 | 76.8±11.3 | 124.1±8.91 | 40.4±7.16 | 36.6±2.38 |
| MCD^{g} | 14,8±0.71^{*} | 4.3±0.36^{*} | 31.5±7.78^{*} | 14.3± 4.05^{*} | 175.0±19.39^{*} | 164.7±18.43^{*} |
| MCD+Cur5 -8 | 15,6±0.74^{* #} | 4.4±0.35^{*} | 42.4±8.51^{*} | 29.3±8.25^{*} | 119.9±3.80 | 88,9±6.39^{#} |
| MCD+EW | 15.2±0.83^{*} | 4.2±0.39^{*} | 30.8±4.54^{*} | 18.5±3.13^{*} | 91.2±13.84^{#} | 68.4±4.17^{#} |
| MCD+EW+ Cur5-8 | 15.3±0.52^{* #} | 4.5±0.37 | 38.6±5.63^{*} | 20.7±2.28^{*} | 89.4±11.87^{#} | 86.1±10.54^{#} |
| BW^{a}, Body weight: TG^{b}, serum triglycerides; TC^{c}, serum total cholesterol: AST^{d}, aspartate transaminase: ALT^{e}, alanine transaminase; Con^{t}, regular diet; MCD^{s}, Methionine choline deficient diet | | | | | | |

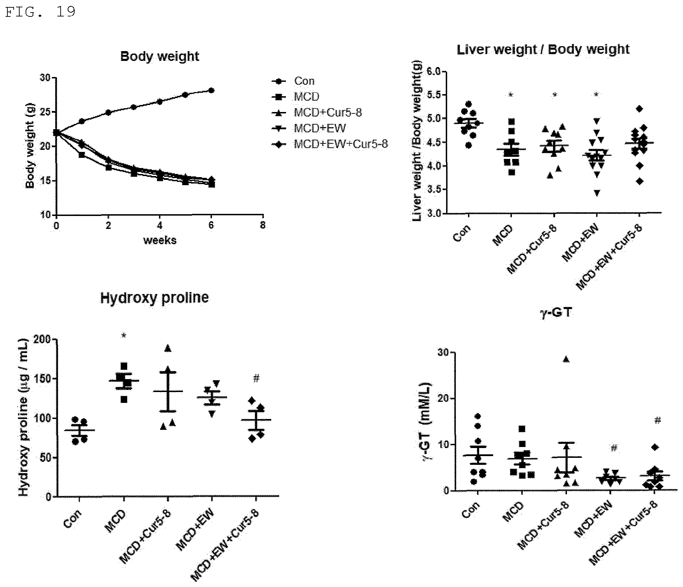

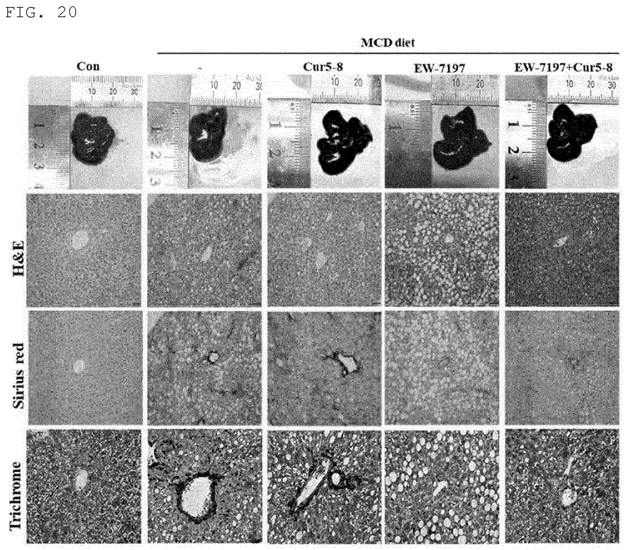

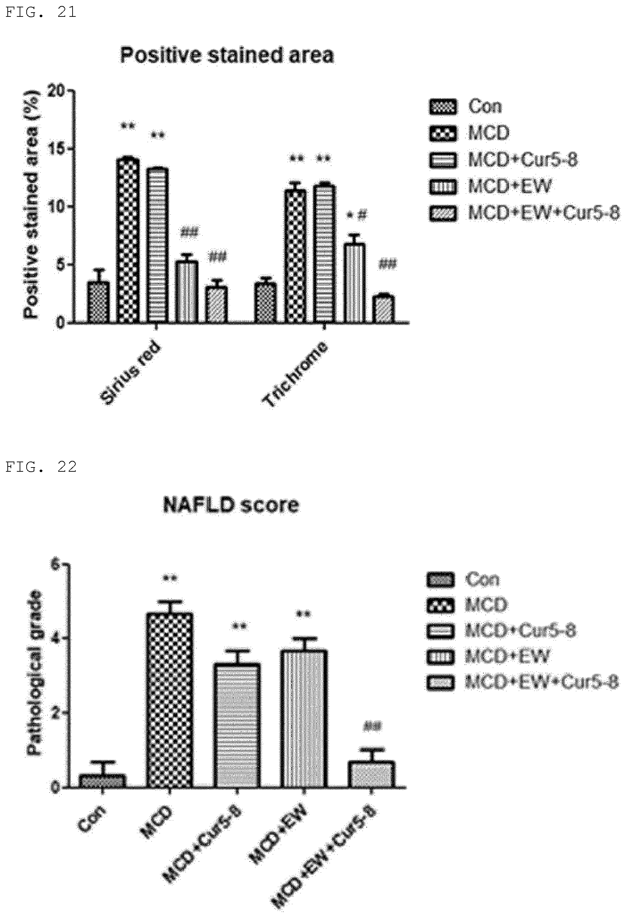

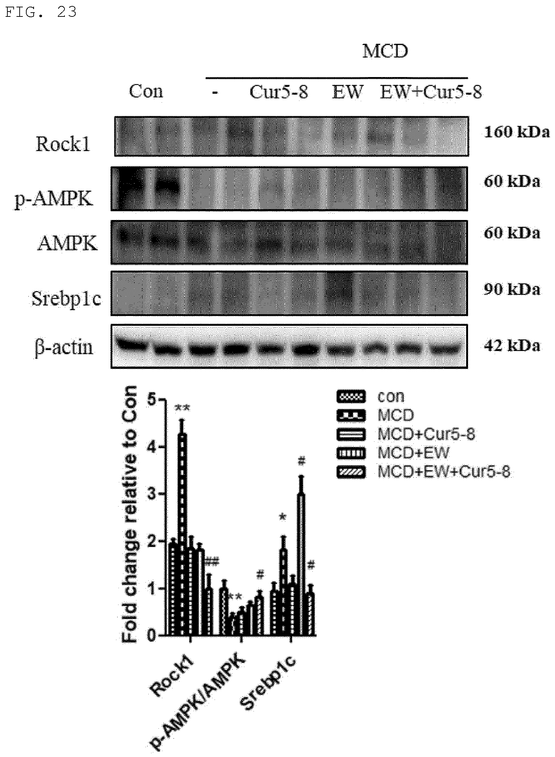

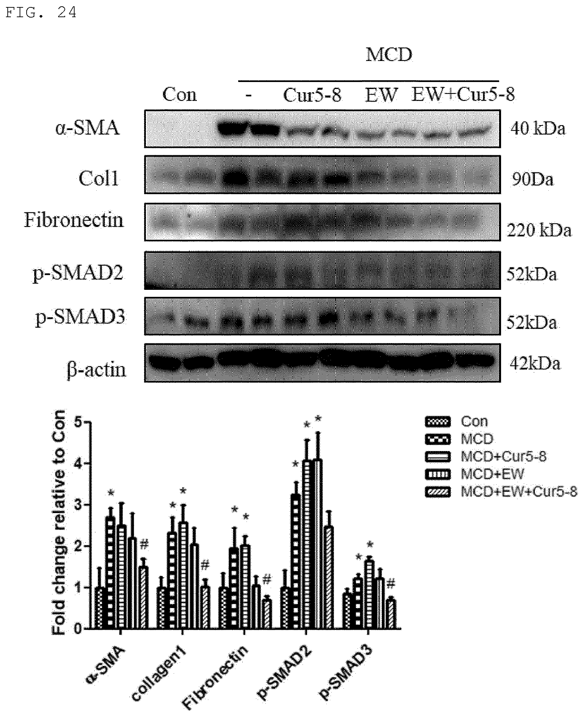

As a result, as shown in Table 1 and FIGS. 19 to 24, it was confirmed that the liver tissue fibrosis lesion occurred when the MCD was fed to C57BL/6 mice. It was confirmed that fibrosis of liver tissues was not improved in the MCD+Cur5-8 administration group, but fibrosis of liver tissues was inhibited in the MCD+EW group and the MCD+EW+Cur5-8 group. Also, it was confirmed that, when MCD was fed to C57BL/6 mice, fat was accumulated in the liver tissues. It was confirmed that fat accumulation in the liver tissues was not inhibited in the MCD+EW group, but fat accumulation in the liver tissues was reduced in the MCD+Cur5-8 group and the MCD+EW+Cur5-8 group.

As a result, it was confirmed that the MCD+EW+Cur5-8 group showed significantly better non-alcoholic steatohepatitis amelioration effect compared to the MCD+EW group and the MCD+Cur5-8 group.

### <Experimental Example 2-2> Confirmation of Inhibiting Liver Fibrosis and Fat Accumulation of Curcumin Derivative and Low-Concentration TGF-β Receptor Inhibitor in Animal Model in which Steatohepatitis was Induced

In order to check the effect of inhibiting fibrosis in liver tissues and inhibiting fat accumulation in liver tissues in case of co-administration of the curcumin derivative (Cur5-8) and the low-concentration TGF-β receptor inhibitor (EW-7197), an animal model in which steatohepatitis was induced was manufactured by the method described in Example 2-2, and the curcumin derivative (CurS-8) and the high-concentration TGF-β receptor inhibitor (5 mg/kg EW-7197) were co-administered. Thereafter, the physiological changes were checked by the method described in Example 2-3, liver tissue lesion was observed by the method described in Example 2-4, and fibrosis and fat synthesis and degradation in liver tissues were checked by the method described in Example 2-5.

**[Table 2]**

| Group | BW (g) | Liver weight/ Body weights 100(g) | TG (mg/dL) | TC (mg/dL) | AST (IU/L) | ALT (IU/L) |
|---|---|---|---|---|---|---|
| MCD | 14.9±0.44 | 4.2±0.13 | 52.4±11.66 | 8.6±1.01 | 168.9±28.37 | 113.0±18.73 |
| MCD+EW 5 | 14.0±0.17 | 4.1±0.09 | 28.3±4.84 | 9.5±2.37 | 159.6±26.73 | 96.1±17.96 |
| MCD+EW 5+Cur5-8 | 14.7±0.29 | 4.7±0.17^{#} | 41.3±12.76 | 9.8±0.85 | 91.3±22.34^{#} | 52.4±11.86^{#} |

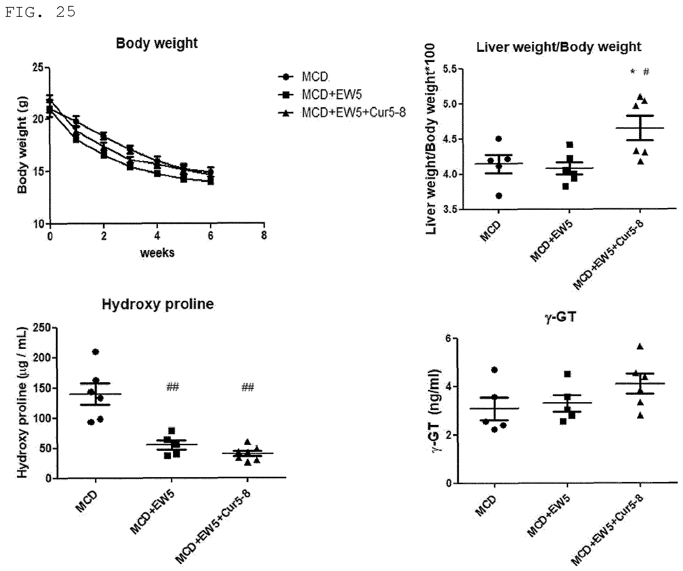

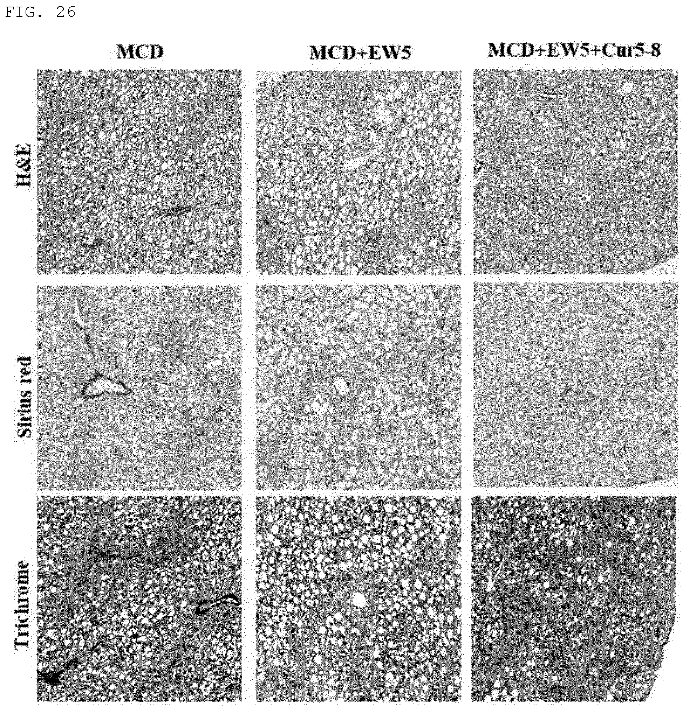

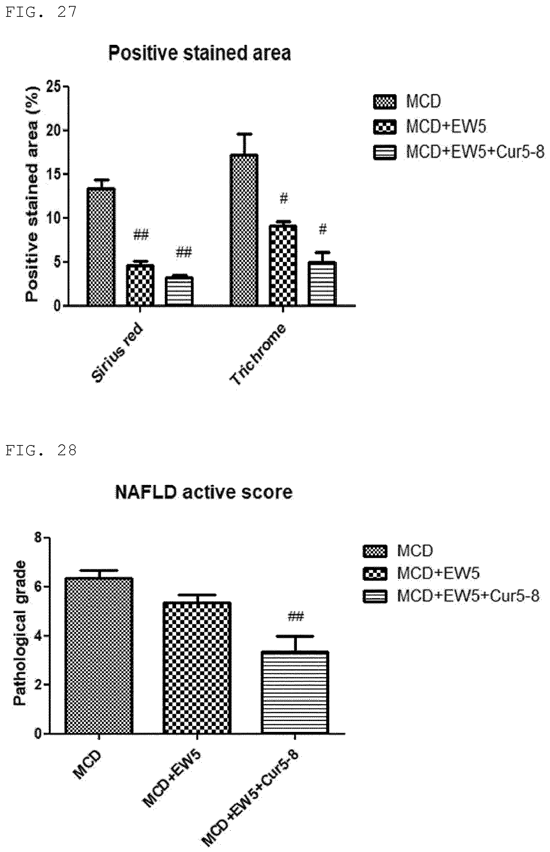

As a result, as shown in Table 2 and FIGS. 25 to 28, even when the low-concentration TGF-β receptor inhibitor was co-administered, fibrosis of liver tissue was inhibited and fat accumulation was reduced similarly to <Experimental Example 2-1>. Accordingly, it was confirmed that the MCD+EW+Cur5-8 group showed significantly better non-alcoholic steatohepatitis amelioration effect compared to the MCD+EW group.

In one example of the present invention, after stimulation of hepatocytes with TGF-β, changes by the co-administration of curcumin derivatives such as curcumin 5-8, dehydrozingerone, or derivatives thereof, with a TGF-β receptor inhibitor such as EW-7197 were checked. As a result, cell transformation by TGF-β was inhibited by EW-7197, adipogenesis increased at this time was inhibited by curcumin derivatives, and thus steatohepatitis prevention, amelioration, treatment effects were significantly better in case of the co-administration than in a case where each was administered alone. Therefore, the curcumin derivative or the pharmaceutically acceptable salts thereof; and the TGF-β receptor inhibitor or the pharmaceutically acceptable salts thereof can be effectively used as active ingredients of the composition for **use in preventing or treating** a metabolic liver disease.

### [Industrial Applicability]

According to the present invention, when a curcumin derivative and a TGF-β receptor inhibitor were co-administered, effect of preventing and treating steatohepatitis more excellent than in a case where the curcumin derivative or the TGF-β receptor inhibitor were administered alone as in the related art was confirmed, and thus the curcumin derivative or the pharmaceutically acceptable salts thereof; and the TGF-β receptor inhibitor or the pharmaceutically acceptable salts thereof can be used as active ingredients of a pharmaceutical composition for use in preventing, ameliorating, or treating a metabolic liver disease including steatohepatitis.

## Claims

1. A pharmaceutical composition for use in preventing or treating a metabolic liver disease, comprising:
a curcumin derivative or pharmaceutically acceptable salts thereof; and a TGF-β receptor inhibitor or pharmaceutically acceptable salts thereof as active ingredients,
wherein the curcumin derivative is at least one selected from the group consisting of N-{3-[(2E)-3-(4-hydroxy-3-methoxyphenyl)prop-2-enoyl]phenyl}-2-methylpropanamide, 4,4'-(3,5-pyridinediyldi-2,1-ethynediyl)bis(2-methoxyphenol), 2,6-dichloro-N-{3-[(2E)-3-(4-hydroxy-3-methoxyphenyl)-2-propenoyl]phenyl}benzamide, dehydrozingerone (DHZ), 4-(3,4-dimethoxyphenyl)-3-buten-2-one (DHZ103), and 1-(4-chlorophenyl)-3-phenyl-1,3-propanedione (DHZ176).

2. The pharmaceutical composition for use in preventing or treating a metabolic liver disease according to Claim 1,
wherein the composition inhibits TGF-β receptor AKL4 (Activin receptor-like kinase 4) or AKL5 (Activin receptor-like kinase 5).

3. The pharmaceutical composition for use in preventing or treating a metabolic liver disease according to Claim 1,
wherein the TGF-β receptor inhibitor is N-(2-fluorophenyl)-5-(6-methyl-2-pyridinyl)-4-[1,2,4]triazolo[1,5-a]pyridin-6-yl-1H-imidazole-2-methanamine (EW-7197).

4. The pharmaceutical composition for use in preventing or treating a metabolic liver disease according to Claim 1,
wherein a molar ratio between the curcumin derivative and the TGF-β receptor inhibitor is 1:15 to 15:1.

5. The pharmaceutical composition for use in preventing or treating a metabolic liver disease according to Claim 1,
wherein the metabolic liver disease is at least one selected from the group consisting of hepatic steatosis, liver fibrosis, and steatohepatitis.

6. The pharmaceutical composition for use in preventing or treating a metabolic liver disease according to Claim 5,
wherein the steatohepatitis is non-alcoholic steatohepatitis.

7. The pharmaceutical composition for use in preventing or treating a metabolic liver disease according to any one of Claims 1 to 6,
wherein the pharmaceutical composition for use in preventing or treating a metabolic liver disease is included in an oral pharmaceutical preparation.

8. The pharmaceutical composition for use in preventing or treating a metabolic liver disease according Claim 7,
wherein the oral pharmaceutical preparation is formulated into at least one selected from the group consisting of tablets, granules, pills, powders, capsules, and solutions.

9. A combination kit for use in treating a metabolic liver disease, the kit comprising a preparation containing a curcumin derivative or pharmaceutically acceptable salts thereof; and a TGF-β receptor inhibitor or pharmaceutically acceptable salts thereof as active ingredients in combination,
wherein the curcumin derivative is at least one selected from the group consisting of N-{3-[(2E)-3-(4-hydroxy-3-methoxyphenyl)prop-2-enoyl]phenyl}-2-methylpropanamide, 4,4'-(3,5-pyridinediyldi-2,1-ethynediyl)bis(2-methoxyphenol), 2,6-dichloro-N-{3-[(2E)-3-(4-hydroxy-3-methoxyphenyl)-2-propenoyl]phenyl}benzamide, dehydrozingerone (DHZ), 4-(3,4-dimethoxyphenyl)-3-buten-2-one (DHZ103), and 1-(4-chlorophenyl)-3-phenyl-1,3-propanedione (DHZ176).

## Patentansprüche

1. Ein Arzneimittel zur Verwendung bei der Vorbeugung oder Behandlung einer metabolischen Lebererkrankung, umfassend:
ein Curcuminderivat oder pharmazeutisch verträgliche Salze davon; und einen TGF-β-Rezeptor-Inhibitor oder pharmazeutisch verträgliche Salze davon als Wirkstoffe,
wobei das Curcuminderivat mindestens eines, ausgewählt aus der Gruppe bestehend aus N-{3-[(2E)-3-(4-Hydroxy-3-methoxyphenyl)prop-2-enoyl]phenyl}-2-methylpropan-amid, 4,4'-(3,5-Pyridindiyl-di-2,1-ethin-diyl)bis(2-methoxyphenol), 2,6-Dichlor-N-{3-[(2E)-3-(4-hydroxy-3-methoxyphenyl)-2-propenoyl]phenyl}benzamid, Dehydrozingeron (DHZ), 4-(3,4-Dimethoxyphenyl)-3-buten-2-on (DHZ103) und 1-(4-Chlorphenyl)-3-phenyl-1,3-propandion (DHZ176), ist.

2. Das Arzneimittel zur Verwendung bei der Vorbeugung oder Behandlung einer metabolischen Lebererkrankung gemäß Anspruch 1,
wobei die Zusammensetzung den TGF-β-Rezeptor ALK4 (Activin-Rezeptor-ähnliche Kinase 4) oder ALK5 (Activin-Rezeptor-ähnliche Kinase 5) hemmt.

3. Das Arzneimittel zur Verwendung bei der Vorbeugung oder Behandlung einer metabolischen Lebererkrankung gemäß Anspruch 1,
wobei der TGF-β-Rezeptor-Inhibitor N-(2-Fluorphenyl)-5-(6-methyl-2-pyridinyl)-4-[1,2,4]triazolo[1,5-a]pyridin-6-yl-1H-imidazol-2-methanamin (EW-7197) ist.

4. Das Arzneimittel zur Verwendung bei der Vorbeugung oder Behandlung einer metabolischen Lebererkrankung gemäß Anspruch 1,
wobei ein Molverhältnis zwischen dem Curcuminderivat und dem TGF-β-Rezeptor-Inhibitor 1:15 bis 15:1 beträgt.

5. Das Arzneimittel zur Verwendung bei der Vorbeugung oder Behandlung einer metabolischen Lebererkrankung gemäß Anspruch 1,
wobei die metabolische Lebererkrankung mindestens eine, ausgewählt aus der Gruppe bestehend aus hepatischer Steatose, Leberfibrose und Steatohepatitis, ist.

6. Das Arzneimittel zur Verwendung bei der Vorbeugung oder Behandlung einer metabolischen Lebererkrankung gemäß Anspruch 5,
wobei es sich bei der Steatohepatitis um eine nichtalkoholische Steatohepatitis handelt.

7. Das Arzneimittel zur Verwendung bei der Vorbeugung oder Behandlung einer metabolischen Lebererkrankung gemäß einem der Ansprüche 1 bis 6,
wobei das Arzneimittel zur Vorbeugung oder Behandlung einer metabolischen Lebererkrankung in einer oralen pharmazeutischen Zubereitung enthalten ist.

8. Das Arzneimittel zur Verwendung bei der Vorbeugung oder Behandlung einer metabolischen Lebererkrankung gemäß Anspruch 7,
wobei die orale pharmazeutische Zubereitung in mindestens eines, ausgewählt aus der Gruppe bestehend aus Tabletten, Granulaten, Pillen, Pulvern, Kapseln und Lösungen, formuliert ist.

9. Ein Kombinationskit zur Verwendung bei der Behandlung einer metabolischen Lebererkrankung, wobei das Kit eine Zubereitung, welche ein Curcuminderivat oder pharmazeutisch verträgliche Salze davon enthält; sowie einen TGF-β-Rezeptor-Inhibitor oder pharmazeutisch verträgliche Salze davon als Wirkstoffe in Form einer Kombination, umfasst,
wobei das Curcuminderivat mindestens eines, ausgewählt aus der Gruppe bestehend aus N-{3-[(2E)-3-(4-Hydroxy-3-methoxyphenyl)prop-2-enoyl]phenyl}-2-methylpropan-amid, 4,4'-(3,5-Pyridindiyl-di-2,1-ethin-diyl)bis(2-methoxyphenol), 2,6-Dichlor-N-{3-[(2E)-3-(4-hydroxy-3-methoxyphenyl)-2-propenoyl]phenyl}benzamid, Dehydrozingeron (DHZ), 4-(3,4-Dimethoxyphenyl)-3-buten-2-on (DHZ103) und 1-(4-Chlorphenyl)-3-phenyl-1,3-propandion (DHZ176), ist.

## Revendications

1. Composition pharmaceutique pour une utilisation dans la prévention ou le traitement d'une maladie hépatique métabolique, comprenant :
un dérivé de curcumine ou des sels pharmaceutiquement acceptables de celui-ci ; et un inhibiteur du récepteur de TGF-β ou des sels pharmaceutiquement acceptables de celui-ci, en tant que principes actifs,
dans laquelle le dérivé de curcumine est au moins l'un choisi dans le groupe constitué par le N-{3-[(2E)-3-(4-hydroxy-3-méthoxyphényl)prop-2-énoyl]phényl}-2-méthylpropanamide, le 4,4'-(3,5-pyridinediyldi-2,1-éthynediyl)bis(2-méthoxy-phénol), le 2,6-dichloro-N-{3-[(2E)-3-(4-hydroxy-3-méthoxyphényl)-2-propénoyl]-phényl}benzamide, la déshydrozingérone (DHZ), la 4-(3,4-diméthoxyphényl)-3-butén-2-one (DHZ103), et la 1-(4-chlorophényl)-3-phényl-1,3-propanedione (DHZ176).

2. Composition pharmaceutique pour une utilisation dans la prévention ou le traitement d'une maladie hépatique métabolique selon la revendication 1, dans laquelle la composition inhibe le récepteur de TGF-β ALK4 (kinase 4 analogue au récepteur d'activine) ou ALK5 (kinase 5 analogue au récepteur d'activine).

3. Composition pharmaceutique pour une utilisation dans la prévention ou le traitement d'une maladie hépatique métabolique selon la revendication 1, dans laquelle l'inhibiteur du récepteur de TGF-β est la N-(2-fluorophényl)-5-(6-méthyl-2-pyridinyl)-4-[1,2,4]triazolo[1,5-a]pyridin-6-yl-1H-imidazole-2-méthanamine (EW-7197).

4. Composition pharmaceutique pour une utilisation dans la prévention ou le traitement d'une maladie hépatique métabolique selon la revendication 1, dans laquelle le rapport molaire entre le dérivé de curcumine et l'inhibiteur du récepteur de TGF-β est de 1/15 à 15/1.

5. Composition pharmaceutique pour une utilisation dans la prévention ou le traitement d'une maladie hépatique métabolique selon la revendication 1, dans laquelle la maladie hépatique métabolique est au moins l'une choisie dans le groupe constitué par une stéatose hépatique, une fibrose hépatique, et une stéatohépatite.

6. Composition pharmaceutique pour une utilisation dans la prévention ou le traitement d'une maladie hépatique métabolique selon la revendication 5, dans laquelle la stéatohépatite est une stéatohépatite non alcoolique.

7. Composition pharmaceutique pour une utilisation dans la prévention ou le traitement d'une maladie hépatique métabolique selon l'une quelconque des revendications 1 à 6, dans laquelle la composition pharmaceutique pour une utilisation dans la prévention ou le traitement d'une maladie hépatique métabolique est incluse dans une préparation pharmaceutique à usage oral.

8. Composition pharmaceutique pour une utilisation dans la prévention ou le traitement d'une maladie hépatique métabolique selon la revendication 7, dans laquelle la préparation pharmaceutique à usage oral est formulée en au moins l'un choisi dans le groupe constitué par les comprimés, les granules, les pilules, les poudres, les gélules, et les solutions.

9. Trousse combinée pour une utilisation dans le traitement d'une maladie hépatique métabolique, la trousse comprenant une préparation contenant un dérivé de curcumine ou des sels pharmaceutiquement acceptables de celui-ci ; et un inhibiteur du récepteur de TGF-β ou des sels pharmaceutiquement acceptables de celui-ci, en tant que principes actifs, en combinaison,
dans laquelle le dérivé de curcumine est au moins l'un choisi dans le groupe constitué par le N-{3-[(2E)-3-(4-hydroxy-3-méthoxyphényl)prop-2-énoyl]phényl}-2-méthylpropanamide, le 4,4'-(3,5-pyridinediyldi-2,1-éthynediyl)bis(2-méthoxy-phénol), le 2,6-dichloro-N-{3-[(2E)-3-(4-hydroxy-3-méthoxyphényl)-2-propénoyl]-phényl}benzamide, la déshydrozingérone (DHZ), la 4-(3,4-diméthoxyphényl)-3-butén-2-one (DHZ103), et la 1-(4-chlorophényl)-3-phényl-1,3-propanedione (DHZ176).
